# EUROPÄISCHE PATENTSCHRIFT

(11) **EP 2 164 831 B1**
(45) Veröffentlichungstag und Bekanntmachung des Hinweises auf die Patenterteilung: **17.07.2013**
(21) Anmeldenummer: 08760299.1
(22) Anmeldetag: 30.05.2008
(51) Int. Cl.: C07D 231/14

(54) **VERFAHREN ZUR HERSTELLUNG N-SUBSTITUIERTER (3-DIHALOMETHYL-1-METHYL-PYRAZOL-4-YL)CARBOXAMIDE**
METHOD FOR THE PRODUCTION OF N-SUBSTITUTED (3-DIHALOMETHYL-1-METHYL-PYRAZOLE-4-YL) CARBOXAMIDES
PROCÉDÉ DE PRODUCTION DE (3-DIHALOGÉNOMÉTHYL-1-MÉTHYL-PYRAZOL-4-YL)CARBOXAMIDE N-SUBSTITUÉ

(30) Priorität: 01.06.2007 EP 07109463
(43) Veröffentlichungstag der Anmeldung: 24.03.2010
(73) Patentinhaber: BASF SE, 67056 Ludwigshafen (DE)
(72) Erfinder: ZIERKE, Thomas, 67459 Böhl-Iggelheim (DE); RHEINHEIMER, Joachim, 67063 Ludwigshafen (DE); RACK, Michael, 69214 Eppelheim (DE); SMIDT, Sebastian Peer, 68163 Mannheim (DE); ALTENHOFF, Ansgar Gereon, 69117 Heidelberg (DE); SCHMIDT-LEITHOFF, Joachim, 68165 Mannheim (DE); CHALLAND, Nina, 68165 Mannheim (DE)
(86) Internationale Anmeldenummer: PCT/EP2008/056712
(87) Internationale Veröffentlichungsnummer: WO 2008/145740

(56) Entgegenhaltungen:
- WO-A-92/12970
- WO-A-03/070705
- WO-A-2005/044804
- DE-A1- 3 934 924
- US-A- 5 093 347
- BROUGH, P.A.; ET AL.: BIOORG. MED. CHEM. LETT., Bd. 15, 2005, Seiten 5197-5201, XP005116273

## Beschreibung

Die vorliegende Erfindung betrifft ein Verfahren zur Herstellung N-substituierter (3-Dihalomethylpyrazol-4-yl)carboxamide, speziell N-substituierter (3-Difluormethylpyrazol-4-yl)carboxamide, die zur Herstellung verwendeten Zwischenprodukte sowie Verfahren zu deren Herstellung.

Die WO 92/12970 beschreibt (3-Difluormethyl-1-methylpyrazol-4-yl)carboxamide und deren Verwendung als Fungizide. Die Herstellung erfolgt ausgehend von einem 4,4-Difluoracetessigester, der sukzessive mit Trifluorethylorthoformiat und mit Methylhydrazin umgesetzt wird, wobei man das (3-Difluormethyl-1-methylpyrazol-4-yl)-carboxylat erhält. Dieses wird anschließend zur Carbonsäure verseift und über die Zwischenstufe des entsprechenden Säurechlorids mit einem geeigneten Amin in das entsprechende Amid überführt.

Die US 5,093,347 beschreibt die Herstellung von 1-Methyl-3-difluormethyl-1 H-pyrazol-4-carbonsäurechlorid ausgehend von einem 4,4-Difluoracetessigester, der sukzessive mit Trifluorethylorthoformiat und mit Methylhydrazin umgesetzt wird, wobei man den (3-Difluormethyl-1-methylpyrazol-4-yl)carbonsäureethylester erhält. Dieser wird anschließend zur Carbonsäure verseift und danach in das Säurechlorid überführt.

In Bioorg. Med. Chem. Lett., Bd. 15, 2005, Seiten 5197-5201 beschreiben Paul A. Brough et al 3-(5-Chlor-2,4-dihydroxyphenyl)-pyrazol-4-carboxamide als Inhibitoren des molekularen Chaperons Hsp90. Die Synthese der Pyrazolcarboxamide umfasst u.a. die Lithiierung eines Brompyrazols und anschließende Umsetzung mit Kohlendioxid unter Erhalt der entsprechenden Carbonsäure. Die Carbonsäure wird anschließend nach Aktivierung mit HATU mit einem Amin in das entsprechende Amid überführt.

Die WO 2005/044804 beschreibt Carboxylate fluormethylsubstituierter Heterocyclen, wie unter anderem 3-Difluormethyl-1-methyl-1H-pyrazol-4-carbonsäureethylester, sowie deren Herstellung durch Halogenaustausch an den entsprechenden Carboxylaten chlormethylsubstituierter Heterocyclen. Weiterhin wird die Umsetzung der erhaltenen Verbindungen zu den entsprechenden Carboxamiden beschrieben.

Die unveröffentlichte EP 06123461.3 beschreibt die Herstellung von (3-Difluormethyl-1-methylpyrazol-4-yl)carboxylaten ausgehend von 2-Alkoxymethylen-4,4,4-trihaloacetessigestern durch teilweise Dehalogenierung und Umsetzung mit einem geeigneten Hydrazinderivat. Weiterhin wird die Umsetzung der erhaltenen Verbindungen zu den entsprechenden Carboxamiden beschrieben.

Die aus dem Stand der Technik bekannten Verfahren zur Herstellung N-substituierter (3-Dihalomethyl-pyrazol-4-yl)carboxamide gehen somit alle von den entsprechenden N-substituierten (3-Dihalomethyl-pyrazol-4-yl)carboxylaten aus. Die Herstellung N-substituierter (3-Dihatomethyl-pyrazol-4-yl)carboxylate ist jedoch vergleichsweise aufwendig.

Der vorliegenden Erfindung liegt somit die Aufgabe zugrunde, ein alternatives Verfahren zur Herstellung N-substituierter (3-Dihalomethyl-pyrazol-4-yl)carboxamide bereitzustellen, welches die Nachteile des Stands der Technik vermeidet. Das Verfahren sollte die Bereitstellung der entsprechenden Carboxylate, deren Verseifung und anschließende Überführung in die entsprechenden Carboxamide, durch eine vergleichsweise weniger aufwändige Syntheseroute ersetzen.

Überraschenderweise wurde gefunden, dass diese Aufgabe durch eine Synthese gelöst wird, bei der die bislang nicht bekannten N-substituierten 4-Brom-3-dihalomethylpyrazole der im Folgenden definierten Formel (II) mit Kohlenmonoxid und geeigneten Anilinen in Gegenwart eines Palladiumkatalysators umgesetzt werden. Die Verbindungen der Formel (II) sind ihrerseits beispielsweise durch Halogenierung entsprechender Verbindungen der im Folgenden definierten Formel (IV) zugänglich.

Gegenstand der vorliegenden Erfindung ist somit ein Verfahren zur Herstellung von Verbindungen der Formel (I), 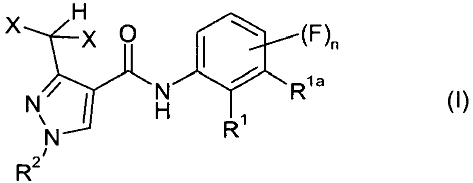 worin
- R¹: für Phenyl oder C₃-C₇-Cycloalkyl steht, die unsubstituiert sind oder 1, 2 oder 3 Substituenten R^{a1} aufweisen, die unabhängig voneinander ausgewählt sind unter Halogen, C₁-C₈-Alkyl, C₁-C₄-Alkoxy, C₁-C₄-Alkylthio, C₁-C₄-Haloalkyl, C₁-C₄-Haloalkoxy, C₁-C₄-Haloalkylthio und C₃-C₇-Cycloalkyl,
- R^{1a}: für Wasserstoff oder Fluor steht, oder R^{1a} zusammen mit R¹ für C₃-C₅-Alkandiyl oder C₅-C₇-Cycloalkandiyl steht, wobei C₃-C₅-Alkandiyl und C₅-C₇-Cycloalkandiyl unsubstituiert sind oder 1, 2 oder 3 Substituenten ausgewählt unter C₁-C₄-Alkyl aufweisen,
- R²: für Wasserstoff, C₁-C₆-Alkyl, C₂-C₆-Alkenyl, C₂-C₆-Alkinyl oder C₁-C₄-Alkoxy-C₁-C₂-alkyl steht,
- X: für F oder Cl steht und
- n: für 0, 1, 2 oder 3 steht;
umfassend
A) die Bereitstellung einer Verbindung der Formel (II) 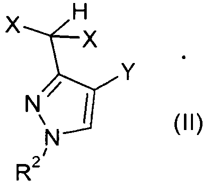 worin
   - X: für F oder Cl steht,
   - Y: für Cl oder Br steht und
   - R²: eine der zuvor gegebenen Bedeutungen besitzt; und
B) die Umsetzung einer Verbindung der Formel (II) mit Kohlenmonoxid und einer Verbindung der Formel (III), 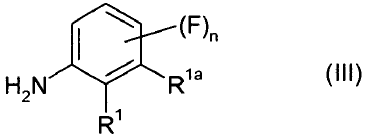 worin R¹, R^{1a} und n eine der zuvor gegebenen Bedeutungen besitzen,
   in Gegenwart eines Palladium-Katalysators.

Ein Spezieller Gegenstand der vorliegenden Erfindung betrifft ein Verfahren zur Herstellung von Verbindungen der Formel (I.1), 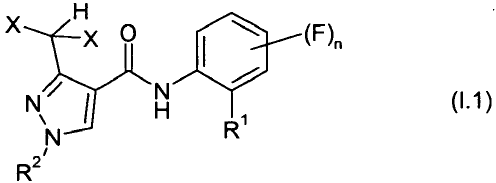 worin
- R¹: für Phenyl oder C₃-C₇-Cycloalkyl steht, die unsubstituiert sind oder 1, 2 oder 3 Substituenten R^{a1} aufweisen, die unabhängig voneinander ausgewählt sind unter Halogen, C₁-C₈-Alkyl, C₁-C₄-Alkoxy, C₁-C₄-Alkylthio, C₁-C₄-Haloalkyl, C₁-C₄-Haloalkoxy, C₁-C₄-Haloalkylthio und C₃-C₇-Cycloalkyl,
- R²: für Wasserstoff, C₁-C₆-Alkyl, C₂-C₆-Alkenyl, C₂-C₆-Alkinyl oder C₁-C₄-Alkoxy-C₁-C₂-alkyl steht,
- X: für F oder Cl steht und
- n: für 0, 1, 2 oder 3 steht;
umfassend
A) die Bereitstellung einer Verbindung der Formel (II) 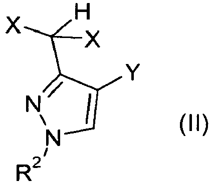 worin
   - X: für F oder Cl steht,
   - Y: für Cl oder Br steht und
   - R²: eine der zuvor gegebenen Bedeutungen besitzt; und
B) die Umsetzung einer Verbindung der Formel (II) mit Kohlenmonoxid und einer Verbindung der Formel (III.1), 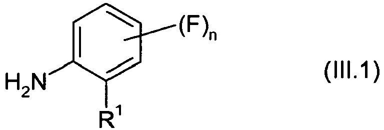 worin R¹ und n eine der zuvor gegebenen Bedeutungen besitzen,
   in Gegenwart eines Palladium-Katalysators,

d. h. ein erfindungsgemäßes Verfahren, worin die Verbindung der Formel (I) ausgewählt ist unter Verbindungen der Formel (I.1), worin R¹, R², X und n unabhängig voneinander eine der für die Verbindungen der Formel (I) gegebenen Bedeutungen aufweisen, und worin die Verbindung der Formel (III) ausgewählt ist unter Verbindungen der Formel (III.1), worin R¹ und n unabhängig voneinander eine der für die Verbindungen der Formel (III) gegebenen Bedeutungen aufweisen.

Die im folgenden bezüglich der Verbindungen der Formeln (I) und (III) und deren Verwendung beschriebenen bevorzugten Ausführungsformen gelten sinngemäß auch für die Verbindungen der Formeln (I.1) und (III.1) und deren Verwendung.

Gegenstand der Erfindung sind auch die Verbindungen der Formel (II), insbesondere Verbindungen der Formel (II), worin X für Fluor steht.

Gegenstand der Erfindung sind auch deren unmittelbare Vorstufe, Verbindungen der Formel (IV) 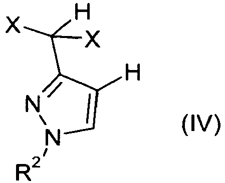 worin R² eine der zuvor gegebenen Bedeutungen besitzt und X für Chlor oder Fluor steht.

Gegenstand der Erfindung sind weiterhin die Verbindung der Formel (VII), (VIII) und (IX), 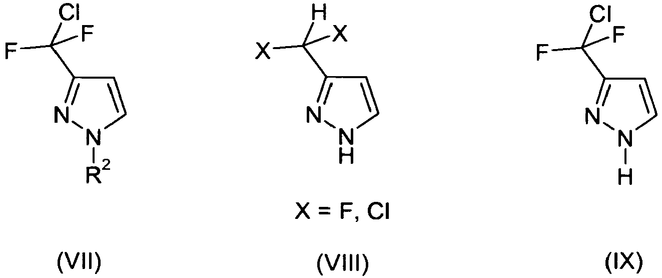 die, wie im Folgenden näher erläutert, in einfacher Weise in die Verbindungen der Formel (IV) überführt werden können.

Weiterer Gegenstand der Erfindung sind Verfahren zur Herstellung der Verbindungen der Formel (II) sowie Verfahren zur Herstellung von deren Synthesevorstufen (IV), (VII), (VIII) und (IX).

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen. Das Präfix Cₓ-C_{y} bezeichnet im jeweiligen Fall die Anzahl möglicher Kohlenstoffatome.

Der Begriff "Halogen" bezeichnet jeweils Fluor, Chlor, Brom oder Iod, speziell Fluor, oder Chlor.

### Beispiele anderer Bedeutungen sind:

Der Begriff "Alkyl", wie in C₁-C₈-Alkyl und in den Begriffen C₁-C₄-Alkoxy, C₁-C₄-Alkoxy-C₁-C₂-Alkyl und C₁-C₄-Alkylthio, verwendet, bezeichnet eine gesättigte, geradkettige oder verzweigte Kohlenwasserstoffgruppe, umfassend speziell 1 bis 8 Kohlenstoffatome bzw. 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, 1-Methylhexyl, 2-Methylhexyl, 3-Methylhexyl, 4-Methylhexyl, 5-Methylhexyl, 1,1-Dimethylpentyl, 1,2-Dimethylpentyl, 1,3-Dimethylpentyl, 1,4-Dimethylpentyl, 2,2-Dimethylpentyl, 2,3-Dimethylpentyl, 2,4-Dimethylpentyl, 3,3-Dimethylpentyl, 3,4-Dimethylpentyl, 1-Ethylpentyl, 2-Ethylpentyl, 1,1,2-Trimethylbutyl, 1,1,3-Trimethylbutyl, 1,2,2-Trimethylbutyl, 1,2,3-Trimethylbutyl, 1,3,3-Trimethylbutyl, 2,2,3-Trimethylbutyl, 2,3,3-Trimethylbutyl, 1-Ethyl-1-methylbutyl, 1-Ethyl-2-methylbutyl, 1-Ethyl-3-methylbutyl, Octyl, 1-Methylheptyl, 2-Methylheptyl, 3-Methylheptyl, 4-Methylheptyl, 5-Methylheptyl, 6-Methylheptyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

Der Begriff " C₂-C₆-Alkenyl" bezeichnet einfach ungesättigte, geradkettige oder verzweigte Kohlenwasserstoffreste mit 2 bis 6 C-Atomen, bevorzugt 2 bis 4, Kohlenstoffatomen und einer C-C- Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Der Begriff "C₂-C₆-Alkinyl" bezeichnet einfach ungesättigte, geradkettige oder verzweigte Kohlenwasserstoffreste mit 2 bis 6 C-Atomen, bevorzugt 2 bis 4, Kohlenstoffatomen und einer C-C-Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, , 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, Dimethyl-3-butinyl, 3,3-Dimethyl-1-butinyl, 3,3-Dimethyl-2-butinyl, Ethyl-2-butinyl, 1-Ethyl-3-butinyl, 2-Ethyl-3-butinyl oder 1-Ethyl-1-methyl-2-propinyl.

Der Begriff "C₁-C₄-Alkoxy" bezeichnet geradkettige oder verzweigte gesättigte Alkylgruppen, umfassend 1 bis 4 Kohlenstoffatome, die über ein Sauerstoffatom gebunden sind. Beispiele umfassen C₁-C₄-Alkoxy wie beispielsweise Methoxy, Ethoxy, OCH₂-C₂H₅, OCH(CH₃)₂, n-Butoxy, OCH(CH₃)-C₂H₅, OCH₂-CH(CH₃)₂ und OC(CH₃)₃.

Der Begriff "C₁-C₄-Alkylthio" bezeichnet geradkettige oder verzweigte gesättigte Alkylgruppen, umfassend 1 bis 4 Kohlenstoffatome, die über ein Schwefelatom gebunden sind. Beispiele umfassen C₁-C₄-Alkylthio wie beispielsweise Methylthio, Ethylthio, SCH₂-C₂H₅, SCH(CH₃)₂, n-Butylthio, SCH(CH₃)-C₂H₅, SCH₂-CH(CH₃)₂ und SC(CH₃)₃.

Der Begriff "C₁-C₄-Haloalkyl", wie hierin und in den Haloalkyleinheiten von C₁-C₄-Haloalkoxy und C₁-C₄-Haloalkylthio verwendet, bezeichnet geradkettige oder verzweigte Alkylgruppen mit 1 bis 4 Kohlenstoffatomen, wobei die Wasserstoffatome dieser Gruppen teilweise oder vollständig durch Halogenatome ersetzt sind, beispielsweise C₁-C₄-Haloalkyl, wie Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl etc..

Der Begriff "C₁-C₄-Haloalkoxy" bezeichnet C₁-C₄-Haloalkylgruppen, wie zuvor definiert, die über ein Sauerstoffatom gebunden sind. Beispiele umfassen Mono-, Di- und Trifluormethoxy, Mono-, Di- und Trichlormethoxy, 1-Fluorethoxy, 1-Chlorethoxy, 2-Fluorethoxy, 2-Chlorethoxy, 1,1-Difluorethoxy, 1,1-Dichlorethoxy, 1,2-Difluorethoxy, 1,2-Dichlorethoxy, 2,2-Difluorethoxy, 2,2-Dichlorethoxy, 2,2,2-Trifluorethoxy, 1,1,2,2-Tetrafluorethoxy, 2,2,2-Trichlorethoxy, 1,1,1,2,3,3-Hexafluorisopropoxy, 1,1,2,3,3,3-Hexafluorisopropoxy, 2-Chlor-1,1,2-trifluorethoxy oder Heptafluorisopropoxy.

Der Begriff "C₁-C₄-Haloalkylthio" bezeichnet C₁-C₄-Haloalkylgruppen, wie zuvor definiert, die über ein Schwefelatom gebunden sind. Beispiele umfassen Mono-, Di- und Trifluormethylthio, Mono-, Di- und Trichlormethylthio, 1-Fluorethylthio, 1-Chlorethylthio, 2-Fluorethylthio, 2-Chlorethylthio, 1,1-Difluorethylthio, 1,1-Dichlorethylthio, 1,2-Difluorethylthio, 1,2-Dichlorethylthio, 2,2-Difluorethylthio, 2,2-Dichlorethylthio, 2,2,2-Trifluorethylthio, 1,1,2,2-Tetrafluorethylthio, 2,2,2-Trichlorethytthio, 1,1,1,2,3,3-Hexafluorisopropylthio, 1,1,2,3,3,3-Hexafluorisopropylthio, 2-Chlor-1,1,2-trifluorethylthio oder Heptafluorisopropylthio.

Der Begriff "C₃-C₇-Cycloalkyl" wie hierin verwendet, beschreibt cyclische Kohlenwasserstoffradikale, umfassend 3 bis 7 Kohlenstoffatome. Beispiele cyclischer Radikale sind Cyclopropyl, Cyclobutyl, Cyclopentyl, Cyclohexyl oder Cycloheptyl.

Der Begriff "Alkandiyl", wie in C₃-C₅-Alkandiyl verwendet, bezeichnet gesättigte, geradkettige Kohlenwasserstoffgruppen, umfassend 3 bis 5 Kohlenstoffatome, die über die beiden terminalen Kohlenstoffatome gebunden sind, wie Propan-1,3-diyl, Butan-1,4-diyl und Pentan-1,5-diyl.

Der Begriff "Cycloalkandiyl", wie in C₅-C₇-Alkandiyl verwendet, bezeichnet gesättigte, cyclische Kohlenwasserstoffgruppen, umfassend 5 bis 7 Kohlenstoffatome als Ringglieder, die über zwei beliebige Kohlenstoffatome gebunden sind, wie Cyclopentan-1,3-diyl, Cyclohexan-1,3-diyl, Cyclohexan-1,4-diyl, Cycloheptan-1,3-diyl und Cycloheptan-1,4-diyl.

In dem erfindungsgemäßen Verfahren zur Herstellung von Verbindungen der Formel (I) aus Verbindungen der Formeln (II) und (III) werden in Schritt B die Verbindungen der Formeln (II) und (III) bevorzugt in einem Molverhältnis (II) : (III) von 0,5 : 1 bis 2 : 1, bevorzugt 0,8 : 1 bis 1,2 : 1 eingesetzt. Insbesondere wird die Verbindung der Formel (III) im leichten Überschuss bezogen auf die Verbindung (II) eingesetzt, d. h. das Molverhältnis (II) : (III) ist < 1, z. B. im Bereich von 0,5 : 1 bis < 1 : 1, insbesondere im Bereich von 0,8 : 1 bis 0,95 : 1.

Geeignete Palladium-Katalysatoren für die Umsetzung der Verbindungen der Formel (II) mit Verbindungen der Formel (III) sind palladiumhaltige Verbindungen, worin Palladium eine Oxidationsstufe von 0 oder 2 aufweist.

Beispiele für palladiumhaltige Verbindungen, die eine Oxidationsstufe von 0 aufweisen sind Palladium(0)ligandkomplexe, wie Palladium(0)tetrakis(triphenylphosphin), Palladium(0)tetrakis(diphenylmethylphosphin) oder Palladium(0)-bis-(DIPHOS), oder metallisches Palladium, das gegebenfalls geträgert ist. Metallisches Palladium ist bevorzugt auf einen inerten Träger, wie Aktivkohle, Aluminiumoxid, Bariumsulfat, Bariumcarbonat oder Calciumcarbonat, aufgezogen. Die Umsetzung in Gegenwart von metallischem Palladium erfolgt bevorzugt in Gegenwart geeigneter Komplexliganden.

Beispiele für palladiumhaltige Verbindungen, die eine Oxidationsstufe von 2 aufweisen sind Palladium(II)ligandkomplexe, wie Palladium(II)acetylacetonat oder Verbindungen der Formel PdX₂L₂ worin X für Halogen steht und L für einen einwertigen Liganden insbesondere für einen Liganden der im folgenden Gezeigten Formeln (A) oder (B), steht, sowie Palladium(II)salze, wie beispielsweise Palladiumacetat oder Palladiumchlorid, bevorzugt Palladiumchlorid.

Bei Verwendung von Palladium(II)salzen erfolgt die Umsetzung bevorzugt in Gegenwart geeigneter Komplexliganden, speziell der Komplexliganden der im Folgenden gezeigten Formeln (A) und (B).

Der Palladium-Katalysator kann in Form eines fertigen Palladium-Komplexes oder als Palladiumverbindung, die unter den Reaktionsbedingungen als Prä-Katalysator zusammen mit geeigneten Liganden die katalytisch aktive Verbindung bildet eingesetzt werden.

Geeignete Komplexliganden für die erfindungsgemäße Umsetzung von Verbindungen der Formel (II) mit Verbindungen der Formel (III) sind beispielsweise mono- oder bidentate Phosphine der im Folgenden gezeigten Formeln (A) und (B) 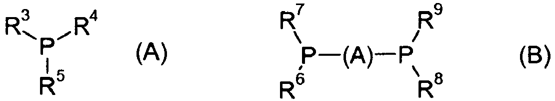 worin R³ bis R⁹ unabhängig voneinander für C₁-C₆-Alkyl, C₅-C₈-Cycloalkyl, Adamantyl, Aryl-C₁-C₂-alkyl oder bevorzugt für Ferrocenyl oder Aryl, das gegebenenfalls durch C₁-C₄-Alkyl oder C₁-C₄-Alkoxy substituiert sein kann, stehen und A für eine lineare, bivalente Kohlenwasserstoffgruppe, vorzugsweise mit 2 bis 5 Kohlenstoffatomen, steht, die unsubstituiert oder gegebenenfalls substituiert ist, wobei die bivalente Kohlenwasserstoffgruppe Teil eines mono- oder bicyclischen Rings sein kann, der wiederum unsubstituiert ist oder weitere Substituenten aufweisen kann.

Speziell steht A in den Verbindungen der Formel (A) und (B) für C₂-C₄-Alkylen, C₀-C₁-Alkylenferrocenyl, 1,1'-Biphenyl-2,2'-diyl oder 1,1'-Binaphtyl-2,2'-diyl steht, wobei die vier zuletzt genannten Gruppen gegebenenfalls durch C₁-C₄-Alkyl oder C₁-C₄-Alkoxy substituiert sein können und wobei C₁-C₄-Alkylen zusätzlich einen oder mehrere Substituenten ausgewählt unter C₃-C₇-Cykloalkyl, Aryl oder Benzyl aufweisen kann. Aryl steht in diesem Zusammenhang für Naphtyl oder gegebenenfalls substituiertes Phenyl. Bevorzugt steht Aryl für Phenyl oder Tolyl, besonders bevorzugt für Phenyl. C₀-C₁-Alkylenferrocenyl steht speziell für Ferrocendiyl, wobei an jedes Cyclopentadien des Ferrocens jeweils eines der beiden Phosphoratome gebunden ist, oder für Methylenferrocenyl, wobei eines der Phosphoratome über die Methylengruppe an ein Cyclopentadien gebunden ist, das zweite Phosphoratom an das selbe Cyclopentadien gebunden ist und die Methylengruppe gegebenenfalls 1 oder 2 weitere Substituenten ausgewählt unter C₁-C₄-Alkyl aufweisen kann.

Bevorzugt werden als Komplexliganden in dem erfindungsgemäßen Verfahren zur Umsetzung von Verbindungen der Formel (II) mit Verbindungen der Formel (III) bidentate Phosphine, wie 1,3-Bis(diphenylphosphino)propan (DPPP), 1,3-Bis(diphenylphosphino)ethan, 1,3-Bis(dicyclohexylphosphino)propan (DCPP), ferrocenylhaltige Phosphine vom Typ JosiPhos, 1,1'-Bis(diphenylphosphino)ferrocen (DPPF) oder 2,2-Dimethyl-1,3-bis(diphenylphosphino)propan und besonders bevorzugt 2,2-Dimethyl-1,3-bis(diphenylphosphino)propan eingesetzt.

Der Palladium-Katalysator wird in dem erfindungsgemäßen Verfahren bevorzugt in einer Menge von 0,01 bis 5 Mol-%, besonders bevorzugt 0,1 bis 1 Mol-%, bezogen auf die eingesetzte Menge des Pyrazols der Formel (II), eingesetzt.

In einer bevorzugten Ausführungsform erfolgt das erfindungsgemäße Verfahren zur Umsetzung von Verbindungen der Formel (II) mit Verbindungen der Formel (III) in Gegenwart einer Hilfsbase.

Geeignete Hilfsbasen sind beispielsweise basische Alkalimetallsalze und tertiäre Amine.

Beispiele für basische Alkalimetallsalze sind Kaliumphosphat, Natriumphosphat, Kaliumcarbonat, Natriumcarbonat, Kaliumacetat oder Natriumacetat. Das Alkalimetallsalz sollte bevorzugt im Wesentlichen wasserfrei sein. Besonders bevorzugt wird trockenes Kaliumcarbonat oder Kaliumphosphat verwendet. Alkalimetallsalze werden in dieser Ausführungsform bevorzugt in einer Menge von wenigstens einem, besonders bevorzugt 1 bis 4 und speziell etwa 2 Moläquivalenten bezogen auf die eingesetzte Menge der Pyrazolverbindung der Formel (II) verwendet.

Geeignete tertiäre Amine sind beispielsweise Tri(C₁-C₆-alkyl)amine, wie Trimethylamin, Triethylamin oder Di-isopropylethylamin, N-Methylpiperidin, Pyridin, substituierte Pyridine wie 2,4,6-Trimethylpyridin (Collidin), 2,6-Dimethylpyridin (Lutidin), 2-Methylpyridin, (α-Picolin), 3-Methylpyridin (β-Picolin), 4-Methylpyridin (γ-Picolin) und 4-Dimethylaminopyridin sowie bicyclische Amine, wie 1,4-Diazabicyclo[2.2.2]octan, 1,8-Diazabicyclo[5.4.0]undec-7-en oder 1,5-Diazabicyclo[4.3.0]non-5-ene. Besonders bevorzugt werden Triethylamin ,Pyridin oder 1,8-Diazabicyclo[5.4.0]undec-7-en verwendet. Tertiäre Amine können in einer Menge von 0,1 bis 4 Moläquivalenten bezogen auf die eingesetzte Menge der Pyrazolverbindung der Formel (II) verwendet.

In einer bevorzugten Ausführungsform des erfindungsgemäßen Verfahrens erfolgt die Umsetzung einer Verbindung der Formel (II) mit einer Verbindung der Formel (III) in Gegenwart wenigstens eines tertiären Amins und wenigstens eines Alkalimetallsalzes.

Das Alkalimetallsalz wird in dieser Ausführungsform bevorzugt in einer Menge von 1 bis 4 und speziell etwa 2 Moläquivalenten bezogen auf die eingesetzte Menge der Pyrazolverbindung der Formel (II) verwendet. Das tertiäre Amin wird in dieser Ausführungsform bevorzugt in einer Menge von 0,1 bis 4, bevorzugt 0,2 bis 0,7 Moläquivalenten, bezogen auf die eingesetzte Menge der Pyrazolverbindung der Formel (II) verwendet.

Die Hilfsbase wird in dieser Ausführungsform bevorzugt in einer Gesamtmenge von 2 bis 5 Moläquivalente, bezogen auf die eingesetzte Menge der Pyrazolverbindung der Formel (II) verwendet.

Die Umsetzung der Verbindung der Formel (II) mit einer Verbindung der Formel (III) erfolgt vorzugsweise in einem organischen Lösungsmittel. Geeignete Lösungsmittel für die Umsetzung von Verbindungen der Formel (II) mit Verbindungen der Formel (III) sind polare Lösungsmittel, beispielsweise Amide wie Dimethylformamid, Dimethylacetamid, oder N-Methylpyrrolidon, Harnstoffe wie 1,3-Dimethyl-2-imidazolidinon (DMEU) oder 1,4-Dimethylhexahydro-2-pyrimidinon (DMPU), Ether wie Tetrahydrofuran (THF) und 1,4-Dioxan, Sulfolan, Dimethylsulfoxid (DMSO) oder Nitrile wie Acetonitril oder Propionitril, sowie Gemische dieser Lösungsmittel. Bevorzugt werden Nitrile wie insbesondere Acetonitril verwendet. Das verwendete Lösungsmittel ist vorzugsweise im Wesentlichen wasserfrei, d.h. das Lösungsmittel weist einen Wassergehalt von weniger als 1000 ppm und insbesondere nicht mehr als 100 ppm auf.

Die Umsetzung von Verbindungen der Formel (II) mit Verbindungen der Formel (III) wird in dem erfindungsgemäßen Verfahren bevorzugt bei einer Temperatur von 100 bis 150 besonders bevorzugt bei einer Temperatur von 110 bis 130 durchgeführt.

Der CO-Partialdruck bei der Umsetzung von Verbindungen der Formel (II) mit Verbindungen der Formel (III) liegt bevorzugt in einem Bereich von 0,9 bis 100 bar, besonders bevorzugt in einem Bereich von 2 bis 20 bar und speziell in einem Bereich von 5 bis 10 bar.

Die Aufarbeitung der bei der Umsetzung von Verbindungen der Formel (II) mit Verbindungen der Formel (III) erhaltenen Reaktionsgemische erfolgt in der Regel wässrig, d. h. das erhaltene Reaktionsgemisch wird mit Wasser oder einer wässrigen Lösung in Kontakt gebracht. Nach Ansäuern der so erhaltenen wasserhaltigen Reaktionsgemische lassen sich die Verbindungen der Formel (I) in der Regel durch Extraktion mit einem organischen Lösungsmittel und anschließendem Entfernen des organischen Lösungsmittels isolieren. Gegebenenfalls kann es vorteilhaft sein, speziell bei Verwendung von mit Wasser mischbaren Lösungsmitteln für die Umsetzung, das Lösungsmittel vor der Extraktion wenigstens teilweise zu entfernen, beispielsweise destillativ.

In einer bevorzugten Ausführungsform des erfindungsgemäßen Verfahrens wird eine Verbindung der Formel (II), worin R² für C₁-C₆-Alkyl, C₂-C₆-Alkenyl, C₂-C₆-Alkinyl oder C₁-C₄-Alkoxy-C₁-C₂-alkyl steht, unter den zuvor genannten Bedingungen mit einer Verbindung der Formel (III) zu einer Verbindung der Formel (I) umgesetzt.

In einer weiteren bevorzugten Ausführungsform des erfindungsgemäßen Verfahrens wird eine Verbindung der Formel (II), worin Y für Br steht, unter den zuvor genannten Bedingungen mit einer Verbindung der Formel (III) zu einer Verbindung der Formel (I) umgesetzt.

Das erfindungsgemäße Verfahren eignet sich speziell zur Herstellung von Verbindungen der Formeln (I.a), (I.b) und (I.c).

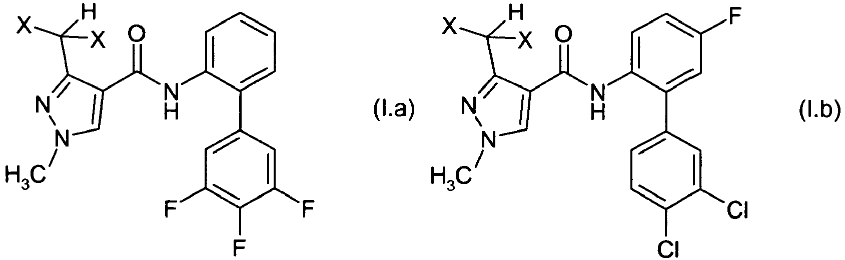

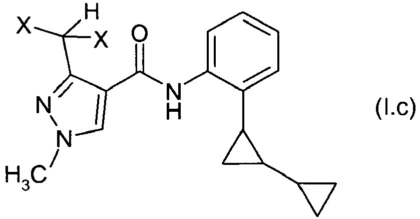

Das erfindungsgemäße Verfahren eignet sich ebenso zur Herstellung von Verbindungen der Formeln (I.e).

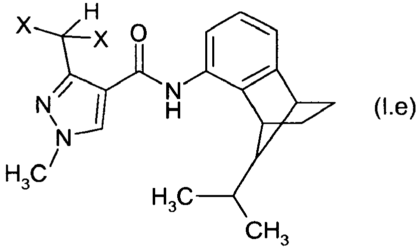

Ein bevorzugter Gegenstand der vorliegenden Erfindung betrifft Verfahren zur Herstellung von Verbindungen der allgemeinen Formel (I) bzw. (I.a), (I.b) und (I.c) bzw. (I.e), worin X für Fluor steht.

Die zur Herstellung von Verbindungen (I) eingesetzten Verbindungen der Formel (II) sind beispielsweise durch die folgenden Methoden herstellbar.

In einer ersten bevorzugten Ausführungsform umfasst die Bereitstellung einer Verbindung der Formel (II), worin Y für Br oder Cl steht,
A.1) die Bereitstellung einer Verbindung der Formel (IV) 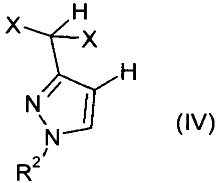 worin
   - X: für Chlor oder Fluor steht und
   - R²: eine der zuvor gegebenen Bedeutungen besitzt,
A.2) gegebenenfalls für den Fall, dass X in der Verbindung der Formel (IV) für Chlor steht, einen Halogenaustausch gegen Fluor und
A.3) die Chlorierung oder Bromierung einer Verbindung der Formel (IV).

Bevorzugt wird man die Bromierung der Verbindungen der Formel (IV) mit Brom (Br₂) in einem inerten Lösungsmittel, z. B. in einem halogenierten Kohlenwasserstoff wie Dichlormethan, durchführen. Die Reaktionstemperatur liegt hierbei vorzugsweise im Bereich von - 5 bis 50 °C und insbesondere bei Raumtemperatur. Weitere Reaktionsbedingungen, die ebenso zielführend sind, sind dem Fachmann bekannt.

Ebenfalls bevorzugt kann man die Bromierung von Verbindungen der Formel (IV) mit N-Bromsuccinimid (NBS) oder 1,3-Dibrom-5,5-dimethylhydantion (DDH) und speziell mit NBS, durchführen. Bevorzugt wird man die Chlorierung von Verbindungen der Formel (IV) mit N-Chlorsuccinimid (NCS). Geeignete Lösungsmittel hierfür sind insbesondere polare Lösungsmittels, wie Dimethylformamid, N-Methylpyrrolidinon, 1,3-Dimethyl-2-imidazolidinon, Dimethylacetamid, Dimethylethylenharnstoff, Dimethylpropylenharnstoff (DMPU) oder Tetramethylharnstoff oder Gemische dieser Lösungsmittel. Die Reaktionstemperatur liegt üblicherweise in einem Bereich von -10 bis 30°C.

Bevorzugt erfolgt die Chlorierung oder Bromierung an einer Verbindung der Formel (IV), worin X für Fluor steht.

Überraschenderweise wurde nun gefunden, dass die Verbindungen der Formel (IV), die bei ihrer Herstellung häufig als schwer zu trennende Gemische mit den jeweiligen Isomeren der Formel (iso-IV) anfallen, 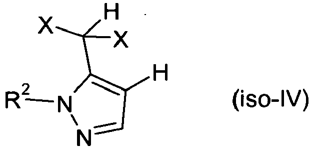 gegenüber einer Chlorierung oder Bromierung reaktiver sind als die entsprechenden Verbindungen der Formel (iso-IV). In der Formel (iso-IV) haben X und R² die für Formel (IV) gegebene Bedeutung. Die unterschiedliche Reaktivität ermöglicht es, in Gemischen der Verbindungen der Formeln (IV) und (iso-IV), das gewünschte Isomer selektiv zu chlorieren oder zu bromieren, wobei ein Gemisch der Verbindungen (II) und (iso-IV) erhalten wird, das sich sehr viel leichter auftrennen lässt als das Gemisch der Verbindungen (IV) und (iso-IV).

In einer speziellen Ausführungsform des erfindungsgemäßen Verfahrens wird man daher die Verbindung der Formel (IV) in Form eines Gemischs mit dem entsprechenden 5-Isomer der Formel (iso-IV) bereitstellen und nach erfolgter Chlorierung oder Bromierung der Verbindung der Formel (IV) die Verbindung der Formel (II) von der unumgesetzten Verbindung der Formel (iso-IV) trennen. Geeignete Trennverfahren sind beispielsweise fraktionierende Destillation oder Rektifikation. Durch diese Vorgehensweise lassen sich vorteilhafterweise hohe Ausbeuteverluste bei der Trennung der Verbindung der Formel (IV) von den Verbindungen der Formel (iso-IV) vermeiden. Hierbei hat es sich als vorteilhaft erwiesen, wenn man die Bromierung der Verbindungen der Formel (IV) mit Brom (Br₂) oder NBS bzw. die Chlorierung mit NCS, wie zuvor beschrieben, durchführt. Die beschriebene Maßnahme eignet sich insbesondere zur Trennung von Gemischen, worin X für Fluor steht.

Im Rahmen der vorliegenden Erfindung wird die Überführung einer dichlormethylsubstituierten Verbindung in die entsprechende difluormethylsubstituierte Verbindung in Gegenwart eines Fluorierungsmittels als Halogenaustausch bezeichnet. Als Fluorierungsmittel eignen sich grundsätzlich alle für Halogenaustauschreaktionen üblicherweise verwendeten Fluorierungsmittel. Der Halogenaustausch erfolgt in dieser Ausführungsform bevorzugt durch Umsetzung mit einem Fluorierungsmittel, das ausgewählt ist unter Alkalifluoriden, Kobalt(III)fluorid, Antimonfluorid, Molybdänfluorid, Fluorwasserstoff, Fluorwasserstoff/Pyridin-Gemischen, tertiären Ammoniumhydrofluoriden und Trialkylaminhydrofluoriden der allgemeinen Formel n*HF/N(C₁-C₄-Alkyl)₃, wobei n für 1, 2 oder 3 steht, vorgenommen wird. Besonders bevorzugt erfolgt der Halogenaustausch durch Umsetzung mit Trialkylaminhydrofluoriden, wie Triethylamin Trishydrofluorid, tri-n-Butylamin-Trishydrofluorid, und ganz besonders bevorzugt mit Triethylamin-Trishydrofluorid.

Das Fluorierungsmittel wird üblicherweise in einem Molmengen-Verhältnis von Fluorid-Äquivalenten pro zu ersetzendem Chloratom im Bereich von 1 : 1 bis 3 : 1 eingesetzt. Bevorzugt wird das Fluorierungsmittel in einem Molmengen-Verhältnis im Bereich von 1 :1 bis 1,5 : 1 eingesetzt.

Die Halogenaustausch-Reaktion findet vorzugsweise bei einer Temperatur im Bereich von 70 bis 180 °C, insbesondere bei einer Temperatur im Bereich von 80 bis 160 °C statt.

Die Halogenaustausch-Reaktion kann bei Normaldruck oder im Autoklaven unter Eigendruck erfolgen. Bevorzugt liegt der Druck in einem Bereich von 0,1 bis 50 bar, speziell im Bereich von 1 bis 10 bar.

Die erfindungsgemäße Halogenaustausch-Reaktion kann gegebenenfalls in Anwesenheit eines Verdünnungsmittels durchgeführt werden. Vorzugsweise wird man hierfür Nitrile, wie Acetonitril, halogenierte Kohlenwasserstoffe, wie Chlorbenzol, Dichlorbenzol, Dichlormethan, Chloroform, Tetrachlormethan, Dichlorethan, Trifluorchlormethan, 1,1,2-Trifluor-1,2,2-trichlorethan oder Trichlorethan, Kohlenwasserstoffe, wie Petrolether, Hexan, Heptan, Cyclohexan, Methylcyclohexan, Benzol, Toluol, Xylol oder Decalin, Ether, wie Diisopropylether, Methyl-tert-butylether, Methyl-tertamylether, Dioxan, Tetrahydrofuran, 1,2-Dimethoxyethan, 1,2-Diethoxyethan oder Anisol, oder Diethylglykol verwenden.

Die Verbindungen der Formel (IV) sind neu und als Zwischenprodukte von Verbindungen der Formel (II) und folglich auch von Verbindungen der Formel (I) geeignet. Demzufolge sind Verbindungen der Formel (IV) ebenfalls Gegenstand der vorliegenden Erfindung. Hierunter sind Verbindungen der allgemeinen Formel (IV) bevorzugt, worin X für Fluor steht. Verbindungen der Formel (IV) worin X für Chlor steht, sind jedoch ebenso Gegenstand der vorliegenden Erfindung.

Geeignete Methoden zur Bereitstellung von Verbindungen der Formel (IV) sind beispielsweise die im Folgenden gezeigten Verfahren.

In einer ersten erfindungsgemäßen Ausführungsform umfasst die Bereitstellung einer Verbindung der Formel (IV) die folgenden Schritte:
A.1.1a) Bereitstellung einer Verbindung der Formel (V) 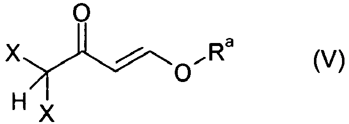 worin
   - R^{a}: C₁-C₆-Alkyl oder C₁-C₆-Alkenyl und
   - X: für Cl oder Fluor steht und
A.1.2a) Umsetzung einer Verbindung der Formel (V) mit einer Hydrazinverbindung der Formel R²HN-NH₂, worin R² eine der zuvor gegebenen Bedeutungen besitzt.

Die Bereitstellung einer Verbindung der Formel (V), worin X für Chlor steht, durch Umsetzung von Dichloracetylchlorid mit Ethylvinylether ist grundsätzlich bekannt (Effenberg, Chem. Ber. 1982,115, 2766). In Abhängigkeit von den Reaktionsbedingungen führt diese Umsetzung zu einer Vielzahl von Nebenprodukten. Im Rahmen der vorliegenden Erfindung haben sich die im Folgenden genannten Reaktionsbedingungen als besonders geeignet erwiesen. Dichloracetylchlorid wird mit Ethylvinylether unter Rückflussbedingungen, d. h. bei einer Temperatur von etwa 38 °C bei Normaldruck, umgesetzt. Alternativ kann die Umsetzung auch zunächst unter Kühlung, d. h. bevorzugt bei -5 bis 10 °C durchgeführt werden und anschließend bei höherer Temperatur vervollständigt werden. Die Bereitstellung von Verbindungen der Formel (V) ist ebenso ausgehend von weiteren Alkylvinylethern möglich.

Der Alkylvinylether wird im Überschuss, d. h. bevorzugt in einer Menge von 3 bis 15 und besonders bevorzugt in einer Menge 6 bis 10 Moläquivalenten, bezogen auf die umzusetzende Menge an Dichloracetylchlorid, eingesetzt. Der Überschuss an Alkylvinylether wird nach beendeter Reaktion destillativ entfernt. Gegebenenfalls erfolgt die Entfernung des Alkylvinylethers nach Zusatz eines inerten höhersiedenden Lösungsmittels, wie Toluol, durch Koevaporation. Die als Rückstand verbleibende Verbindung der Formel (V) kann wenn nötig durch Destillation aufgereinigt werden. In einer speziellen Ausführungsform erfolgt die weitere Umsetzung mit Methylhydrazin ohne vorherige Isolierung der Verbindung der Formel (V).

Verbindungen der Formel (V), worin X für Fluor steht, lassen sich analog durch Umsetzung von Difluoracetylchlorid mit Ethylvinylether bereitstellen.

Für die anschließende Umsetzung einer Verbindung der Formel (V) mit einer Hydrazinverbindung der Formel R²HN-NH₂, worin R² eine der zuvor gegebenen Bedeutungen besitzt und bevorzugt für Methyl steht, hat es sich, insbesondere hinsichtlich der Regioselektivität der Bildung von Verbindungen der Formel (IV) im Vergleich zu den entsprechenden 5-dihalomethylsubstituierten Isomeren, als vorteilhaft erwiesen, wenn man aromatische Kohlenwasserstoffe, wie Benzol, Toluol, Ethylbenzol, Xylole, Cumol, Chlorbenzol, Nitrobenzol oder tert.-Butylbenzol, oder ein Gemisch dieser Lösungsmittel mit Wasser, als Lösungsmittel verwendet. Die Umsetzung kann wasserfrei durchgeführt werden, d.h. die Konzentration an Wasser, bezogen auf die Gesamtlösungsmittelmenge beträgt weniger als 0,5 Vol.-%. Alternativ kann die Umsetzung in Gegenwart von Wasser erfolgen. Vorzugsweise wird die Wassermenge 30 Vol.-%, insbesondere 15 Vol.-%, bezogen auf die Gesamtmenge an organischem Lösungsmittel + Wasser nicht überschreiten und liegt vorzugsweise im Bereich von 0,5 bis 30 Vol.-%, insbesondere im Bereich von 1 bis 15 Vol.-%, bezogen auf die Gesamtmenge an organischem Lösungsmittel + Wasser.

Vorzugsweise führt man die Umsetzung einer Verbindung der Formel (V) mit einer Hydrazinverbindung der Formel R²HN-NH₂, worin R² eine der zuvor gegebenen Bedeutungen besitzt bei Temperaturen von 60 bis 150 °C durch. In der Regel ist die obere Temperaturgrenze der Siedepunkt des jeweiligen Lösungsmittels bei druckloser Reaktionsführung.

Vorzugsweise setzt man die Hydrazinverbindung der Formel R²HN-NH₂, in wenigstens äquimolaren Mengen oder im Überschuss ein. Vorzugsweise setzt man 1,0 bis 1,2 Mol, insbesondere etwa 1,01 bis 1,1 Mol der Hydrazinverbindung der Formel R²HN-NH₂, speziell Methylhydrazin, je Mol der Verbindung (V) ein.

Bei der Umsetzung der Verbindung (V) mit Methylhydrazin wird man in der Regel die Hydrazinverbindung der Formel R²HN-NH², vorzugsweise als Lösung in dem für die Umsetzung verwendeten organischen Lösungsmittel oder in Wasser, zu der Verbindung der Formel (V) oder zu einer Lösung davon in einem organischen Lösungsmittel oder Lösungsmittel/Wasser-Gemisch geben. Die Zugabe in umgekehrter Reihenfolge oder die zeitgleiche Zugabe der Ausgangsverbindungen ist ebenfalls möglich.

In einer speziellen erfindungsgemäßen Ausführungsform werden die bei der Umsetzung der Verbindung (V) mit einer Hydrazinverbindung der Formel R²HN-NH₂entstehenden flüchtigen Bestandteile kontinuierlich durch Destillation aus dem Reaktionsgemisch entfernt. Das dabei gegebenenfalls gleichzeitig aus dem Reaktionsgemisch entfernte Lösungsmittel, wird kontinuierlich durch die entsprechende Menge an frischem Lösungsmittel ersetzt.

In einer alternativen erfindungsgemäßen Ausführungsform werden die Verbindung der Formel (IV) ausgehend von Verbindungen der Formel (V), durch Umsetzung einer Verbindung der Formel (V), mit Hydrazin zu einer Verbindung der Formel (VIII), 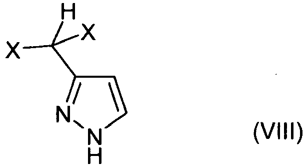 worin X für Cl oder Fluor steht, und anschließender N-Alkylierung der Verbindung der Formel (VIII) bereitgestellt.

In dieser Ausführungsform wird Hydrazin bevorzugt in Form von Hydrazinhydrat verwendet.

Vorzugsweise setzt man Hydrazin, gegebenenfalls als Hydrazinhydrat, in wenigstens äquimolaren Mengen oder im Überschuss ein. Vorzugsweise setzt man 1,0 bis 2,0 Mol, insbesondere etwa 1,2 bis 1,8 Mol Hydrazin je Mol der umzusetzenden Verbindung der Formel (V) ein.

Zur Umsetzung von Verbindungen der Formel (V) mit Hydrazin wird man in der Regel so vorgehen, dass man die Verbindung der Formel (V), vorzugsweise in Form einer Lösung in einem geeigneten organischen Lösungsmittel, zu der Hydrazin-Verbindung, bevorzugt zu einer Lösung von Hydrazinhydrat, gibt. Vorzugsweise legt man Hydrazin als Lösung in einem organischen Lösungsmittel oder Lösungsmittel/Wasser-Gemisch vor. Alternativ kann man auch so vorgehen, dass man Hydrazin, speziell Hydrazinhydrat, vorzugsweise als Lösung in einem organischen Lösungsmittel oder Lösungsmittel/Wasser-Gemisch, zu der Verbindung der Formel (V) oder zu einer Lösung davon in einem organischen Lösungsmittel oder Lösungsmittel/Wasser-Gemisch gibt.

Für die Umsetzung mit Hydrazin, speziell Hydrazinhydrat, geeignete organische Lösungsmittel sind protisch polare Lösungsmittel, z.B. aliphatische Alkohole mit vorzugsweise 1 bis 4 Kohlenstoffatomen, wie Methanol, Ethanol, n-Propanol, Isopropanol, n-Butanol, Isobutanol oder tert.-Butanol, oder Carbonsäuren, wie Essigsäure, aromatische Kohlenwasserstoffe, wie Benzol, Toluol, Xylole, Cumol, Chlorbenzol, Nitrobenzol oder tert.-Butylbenzol, aprotische polare Lösungsmittel, z.B. cyclische oder acyclische Ether wie Diethylether, tert.-Butylmethylether (MTBE), Tetrahydrofuran (THF) oder Dioxan, cyclische oder acyclische Amide, wie Dimethylformamid, Dimethylacetamid, N-Methylpyrrolidon oder Tetramethylharnstoff, oder aliphatische Nitrile wie Acetonitril oder Propionitril, sowie Mischungen der zuvor genannten Lösungsmittel.

Vorzugsweise erfolgt die Umsetzung in einem protisch polaren Lösungsmittel, insbesondere in einem C₁-C₄-Alkohol oder einer Carbonsäure und besonders bevorzugt in Methanol, Ethanol, Acetonitril oder Essigsäure, oder in einer Mischung eines protisch polaren Lösungsmittels mit einem aprotisch polaren Lösungsmittel oder in einem Gemisch dieser Lösungsmittel mit Wasser. Die Umsetzung kann wasserfrei durchgeführt werden, d.h. die Konzentration an Wasser, bezogen auf die Gesamtlösungsmittelmenge beträgt weniger als 0,5 Vol.-%. Vorzugsweise erfolgt jedoch die Umsetzung in Schritt b) in Gegenwart von Wasser. Vorzugsweise wird die Wassermenge 30 Vol.-%, insbesondere 15 Vol.-%, bezogen auf die Gesamtmenge an organischem Lösungsmittel + Wasser nicht überschreiten und liegt vorzugsweise im Bereich von 0,5 bis 30 Vol.-%, insbesondere im Bereich von 1 bis 15 Vol.-% , bezogen auf die Gesamtmenge an organischem Lösungsmittel + Wasser.

Vorzugsweise führt man die Umsetzung bei Temperaturen von -80 bis +100°C durch. In der Regel ist die obere Temperaturgrenze der Siedepunkt des jeweiligen Lösungsmittels bei druckloser Reaktionsführung. Vorzugsweise wird man eine Reaktionstemperatur von 60°C und insbesondere 40°C nicht überschreiten. Häufig wird man die Reaktion aus praktischen Gründen bei Raumtemperatur durchführen.

Die Aufarbeitung der Reaktionsmischung und Gewinnung der Pyrazolverbindung der Formel (VIII) erfolgt in üblicher Weise, beispielsweise durch Entfernen des Lösungsmittels, z.B. durch Destillation oder durch eine wässrig extraktive Aufarbeitung oder durch eine Kombination dieser Maßnahmen. Eine weitere Reinigung kann beispielsweise durch Kristallisation oder durch Chromatographie erfolgen. Häufig fällt das Produkt jedoch bereits in einer Reinheit an, die weitere Reinigungsverfahren überflüssig macht.

Die Verbindungen der Formel (VIII) werden anschließend durch N-Alkylierung in Verbindungen der Formel (IV) überführt.

Geeignete Bedingungen für die N-Alkylierung von Pyrazolen sind an sich bekannt. Für die N-Alkylierung von Verbindungen der Formel (VIII) hat sich insbesondere die Verwendung von Alkylierungsmittel in Gegenwart anorganischer Basen als geeignet erwiesen.

Geeignete Alkylierungsmittel sind beispielsweise Verbindungen der Formel LG-R². In den Alkylierungsmitteln LG-R² kann LG ausgewählt sein unter Halogen oder O-SO₂-Rm, worin R^{m} für C₁-C₄-Alkyl, C₁-C₄-Alkoxy oder Aryl steht, die gegebenenfalls durch Halogen, C₁-C₄-Alkyl oder Halo-C₁-C₄-alkyl substituiert sind. Dabei besitzt R² eine der zuvor gegebenen Bedeutungen und steht insbesondere für C₁-C₆-Alkyl und speziell für Methyl.

Die Alkylierung erfolgt üblicherweise bei Temperaturen im Bereich von -78 °C bis zum Siedepunkt der Reaktionsmischung, bevorzugt von -50 °C bis 65 °C, insbesondere bevorzugt von -30 °C bis 65 °C. In der Regel wird die Umsetzung in einem Lösungsmittel vorgenommen, bevorzugt in einem inerten organischen Lösungsmittel.

Geeignete Lösungsmittel sind unter anderem Toluol, Dichlormethan, Tetrahydrofuran oder Dimethylformamid oder deren Mischungen.

Geeignete Basen sind anorganische Verbindungen wie Alkalimetall- und Erdalkalimetallhydroxide wie Lithiumhydroxid, Natriumhydroxid, Kaliumhydroxid oder Calciumhydroxid, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat. Besonders geeignet sind Alkalimetall- und Erdalkalimetallcarbonate oder Alkalimetall- und Erdalkalimetallhydroxide. Selbstverständlich können auch Mischungen verschiedener Basen verwendet werden.

Die Basen werden im Allgemeinen äquimolar eingesetzt. Sie können auch im Überschuss oder selbst als Lösungsmittel verwendet werden. In einer bevorzugten Ausführungsform des erfindungsgemäßen Verfahrens wird die Base in äquimolarer Menge oder im Wesentlichen äquimolarer Menge zugesetzt.

In einer weiteren bevorzugten Ausführungsform des erfindungsgemäßen Verfahrens handelt es sich bei der N-Alkylierung um eine N-Methylierung. In dieser Ausführungsform sind insbesondere Dimethylsulfat oder Trimethylphosphat als Alkylierungsmittel geeignet.

Der zuvor erläuterte alternative Weg ist insbesondere bevorzugt, wenn X in den Verbindungen der Formel (V) für Fluor steht, wohingegen die zuvor beschriebene Umsetzung von Verbindungen der Formel (V) mit Hydrazinverbindungen der Formel R²HN-NH₂ insbesondere dann bevorzugt ist, wenn X für Chlor steht.

Alternativ können Verbindungen der Formel (IV), worin X für Chlor steht auch durch Umsetzung von 1,1,4-Trichlor-3-buten-2-on mit 1,1-Dimethylhydrazin bereitgestellt werden.

Verbindungen der Formel (IV), worin X für Chlor steht, können wie zuvor gezeigt durch Halogenaustausch in Verbindungen der Formel (IV), worin X für Fluor steht, überführt werden.

Alternativ können Verbindungen der Formel (VIII), worin X für Cl steht, einem Halogenaustausch und einer anschließenden N-Alkylierung, unter Erhalt einer Verbindung der Formel (IV), worin X für Fluor steht, unterworfen werden.

In einer weiteren erfindungsgemäßen Ausführungsform umfasst die Bereitstellung einer Verbindung der Formel (IV) die folgenden Schritte:
A.1.1b) Bereitstellung einer Verbindung der Formel (VI), 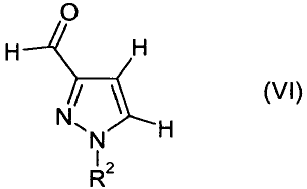 worin R² eine der zuvor gegebenen Bedeutungen besitzt, sowie
A.1.2b) Umsetzung einer Verbindung der Formel (VI) durch Überführung der Carbonylgruppe in eine Dihalomethylgruppe mit einem geeigneten Halogenierungsmittel.

Die Bereitstellung einer Verbindung der Formel (VI) sowie die Überführung einer Carbonylgruppe in eine Dihalomethylgruppe erfolgt bevorzugt unter den zuvor beschriebenen Bedingungen bzw. analog der zuvor für die 4-chlorierten oder 4-bromierten Verbindungen der Formel (VI) beschriebenen Methoden.

In einer weiteren erfindungsgemäßen Ausführungsform umfasst die Bereitstellung einer Verbindung der Formel (IV) die folgenden Schritte:
A.1.1c) Deprotonierung eines Propargylaldehyd-Acetals,
A.1.2c) Umsetzung des deprotonierten Propargylaldehyd-Acetals aus Schritt A.1.1 c mit einer Verbindung der Formel CHX₂Cl, worin X für F oder Cl steht, und
A.1.3c) das anschließende Überführen des Umsetzungsprodukts aus Schritt A.1.2c in eine Verbindung der Formel (IV) mit einer Hydrazinverbindung der Formel R²HN-NH₂, worin R² eine der zuvor gegebenen Bedeutungen besitzt.

Propargylaldehyd Acetale, insbesondere Propargylaldehyddimethylacetal, sind beispielsweise durch Acetalisierung von Acrolein mit Trialkylformiaten, z.B. Trimethylformiat, anschließender Bromaddition und zweifacher HBr-Eliminierung zugänglich (Tetrahedron 2001, 56 (3), 425).

Die Deprotonierung des Propargylaldehyd-Acetals erfolgt bevorzugt durch Umsetzung mit einer geeigneten Base. Geeignete Basen sind beispielsweise Alkyllithium Verbindungen, wie Butyllithium, oder Alkalimetallamide, wie LiNH₂ oder NaNH₂.

Üblicherweise wird man die Deprotonierung in einem geeigneten trockenen Lösungsmittel, bei einer Temperatur von -120 bis -20 °C, bevorzugt -100 bis -50 °C, vornehmen. Trockenes Lösungsmittel bedeutet, dass das Lösungsmittel einen Wassergehalt von weniger als 1000 ppm und insbesondere nicht mehr als 100 ppm aufweist. Beispiele für geeignete Lösungsmittel sind z. B. cyclische oder acyclische Ether wie Diethylether, tert.-Butylmethylether (MTBE), Diisopropylether, Tetrahydrofuran (THF) oder Dioxan, aromatische oder (cyclo)aliphatische Kohlenwasserstoffe wie Toluol, Xylole, Hexan, Cyclohexan und dergleichen, sowie Gemische dieser Lösungsmittel.

Die Umsetzung des deprotonierten Propargylaldehyd-Acetals erfolgt zweckmäßigerweise ohne vorherige Isolierung der deprotonierten Verbindung, unter den zuvor beschriebenen Bedingungen, durch Zugabe einer Verbindung der Formel CHX₂Cl. Die Verbindung CHX₂Cl wird häufig in Form einer Lösung, bevorzugt als Lösung in dem für die Reaktion verwendeten Lösungsmittel, zugegeben. Steht X in der Verbindung der Formel CHX₂Cl für F, wird diese bevorzugt als Gas zugegeben.

Üblicherweise wird man das so erhaltene dihalomethylsubstituierte Propargylaldehyddialkylacetal durch wässrige, extraktive Aufarbeitung isolieren. Eine weitere Aufreinigung des Reaktionsprodukts kann beispielsweise destillativ erfolgen.

Die Umsetzung des dihalomethylsubstituierten Propargylaldehyddialkylacetals zu einer Verbindung der Formel (IV) mit einer Hydrazinverbindung der Formel R²HN-NH₂, speziell mit Methylhydrazin, erfolgt bevorzugt in Gegenwart von konzentrierter Schwefelsäure. Die Schwefelsäure wird dabei in etwa äquimolar verwendet. Vorzugsweise führt man in dieser speziellen Ausführungsform die Umsetzung bei einer Temperaturen von 60 bis 150 °C durch. In der Regel ist die obere Temperaturgrenze der Siedepunkt des verwendeten Lösungsmittels bei druckloser Reaktionsführung. Bevorzugtes Lösungsmittel für diese spezielle Ausführungsform ist Essigsäure (Eisessig). Vorzugsweise setzt man in dieser speziellen Ausführungsform die Hydrazinverbindung der Formel R²HN-NH₂ im Überschuss ein. Vorzugsweise setzt man 1 bis 3 Mol, insbesondere etwa 1,5 bis 2,5 Mol der Hydrazinverbindung je Mol des dihalomethylsubstituierten Propargylaldehyddialkylacetals ein. Besonders bevorzugt wird in dieser speziellen Ausführungsform die Hydrazinverbindung der Formel R²HN-NH₂ und speziell Methylhydrazin in Form einer wässrigen Lösung eingesetzt.

In einer alternativen erfindungsgemäßen Ausführungsform werden die Verbindungen der Formel (IV) ausgehend von einem Propargylaldehyd-Acetal, durch Überführung des Umsetzungsprodukts aus Schritt A.1.2c mit Hydrazin in eine Verbindung der Formel (VIII), wie zuvor definiert, und anschließender N-Alkylierung der Verbindung der Formel (VIII) bereitgestellt.

Bezüglich der Bereitstellung des Umsetzungsprodukts aus Schritt A.1.2c gilt das zuvor für die Bereitstellung einer Verbindung der Formel (V) ausgehend von einem Propargylaldehydacetal Gesagte. Bezüglich der Reaktion des Umsetzungsprodukts mit Hydrazin zu einer Verbindung der Formel (VIII) gilt sinngemäß das zuvor für die Bereitstellung einer Verbindung der Formel (V) aus dem Umsetzungsprodukt Gesagte, wobei an Stelle von Methylhydrazin in Gegenwart von Schwefelsäure zur Bereitstellung einer Verbindung der Formel (VIII) bevorzugt Hydrazinsulfat eingesetzt wird.

Bezüglich der N-Alkylierung einer Verbindung der Formel (VIII) gilt das zuvor Gesagte.

Dieser Weg ist insbesondere bevorzugt, wenn X in dem Umsetzungsprodukt aus Schritt A.1.2c für Fluor steht, wohingegen die zuvor beschriebene Umsetzung des Umsetzungsprodukts aus Schritt A.1.2c mit einer Hydrazinverbindung der Formel R²HN-NH₂ insbesondere dann bevorzugt ist, wenn X für Chlor steht.

In einer weiteren erfindungsgemäßen Ausführungsform umfasst die Bereitstellung einer Verbindung der Formel (IV), worin X für Fluor steht, die folgenden Schritte:
A.1.1d) Bereitstellung einer Verbindung der Formel (VII) 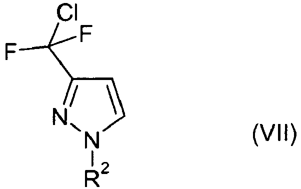 worin R² eine der zuvor gegebenen Bedeutungen besitzt und
A.1.2d) Dechlorierung der Verbindung der Formel (VII).

Methoden zur selektiven Entfernung von Chlor aus einer Difluormethylgruppe, hier als Dechlorierung bezeichnet, sind beispielsweise in Medebielle, Tetrahedron Lett. 2001, 42, 4811 oder Harris, Synth. Comm. 1987, 17, 1587 beschrieben. Beispielsweise können Verbindungen der Formel (VII) in Gegenwart von Reduktionsmitteln, wie Raney-Nickel oder Rongalit (NaO-S(=O)CH₂OH · 2H₂O), zu Verbindungen der Formel (IV), worin X für Fluor steht, umgesetzt werden.

Wie gezeigt, eignen sich die Verbindungen der Formel (VII) vorteilhafterweise für die erfindungsgemäße Bereitstellung von Verbindungen der Formel (IV) und somit für die erfindungsgemäße Bereitstellung von Verbindungen der Formel (I). Demzufolge betrifft ein weiterer Gegenstand der vorliegenden Erfindung Verbindungen der Formel (VII).

Verbindungen der Formel (VII) können beispielsweise durch Umsetzung von 4-Alkoxy-1-chlor-1,1-difluorbut-3-en-2-onen mit einer Hydrazinverbindung der Formel R²HN-NH₂, speziell Methylhydrazin, bereitgestellt werden. 4-Alkoxy-1-chlor-1,1-difluorbut-3-en-2-one können beispielsweise, analog der zuvor beschriebenen Bereitstellung von Verbindungen (V), durch Umsetzung von Chlordifluoracetylchlorid oder Chlordifluoracetanhydrid anstelle von Dihaloacetylchloriden mit Alkylvinylethern, bereitgestellt werden.

Für die Umsetzung der Verbindung der Formel (VII) mit einer Hydrazinverbindung der Formel R²HN-NH₂, speziell Methylhydrazin, hat es sich als besonders vorteilhaft erwiesen, Essigsäure als Lösungsmittel zu verwenden. Die Umsetzung wird in dieser speziellen Ausführungsform bevorzugt wasserfrei durchgeführt, d.h. die Konzentration an Wasser, bezogen auf die Gesamtlösungsmittelmenge beträgt weniger als 0,5 Vol.-%. Unter diesen speziellen Reaktionsbedingungen wird die Umsetzung der Verbindung der Formel (VII) mit einer Hydrazinverbindung der Formel R²HN-NH₂, speziell Methylhydrazin, in der Regel ohne Zufuhr thermischer Energie durchgeführt.

Vorzugsweise setzt man die Hydrazinverbindung der Formel R²HN-NH₂ in wenigstens äquimolaren Mengen oder im Überschuss ein. Vorzugsweise setzt man 1,0 bis 1,5 Mol, insbesondere etwa 1,01 bis 1,2 Mol der Hydrazinverbindung der Formel R²HN-NH₂ je Mol der Verbindung (VII) ein.

Bei der Umsetzung der Verbindung (VII) mit einer Hydrazinverbindung der Formel R²HN-NH₂ wird man in der Regel die Hydrazinverbindung, vorzugsweise als Lösung in dem für die Umsetzung verwendeten organischen Lösungsmittel, zu der Verbindung der Formel (VII), oder zu einer Lösung davon in einem organischen Lösungsmittel, geben.

In einer weiteren erfindungsgemäßen Ausführungsform umfasst die Bereitstellung einer Verbindung der Formel (VIII), worin X für F steht, die Bereitstellung einer Verbindung der Formel (IX) 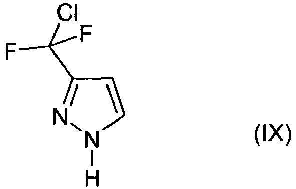 sowie die Dechlorierung der Verbindung der Formel (IX).

Bezüglich der Dechlorierung von Verbindungen der Formel (IX) gilt das zur Dechlorierung von Verbindungen der Formel (VII) Gesagte sinngemäß.

Wie gezeigt eignen sich die Verbindungen der Formel (IX) vorteilhafterweise für die erfindungsgemäße Bereitstellung von Verbindungen der Formel (VIII) und somit für die erfindungsgemäße Bereitstellung von Verbindungen der Formel (I). Demzufolge betrifft ein weiterer Gegenstand der vorliegenden Erfindung Verbindungen der allgemeinen Formel (IX).

Die Bereitstellung von Verbindungen der Formel (IX) kann beispielsweise in Analogie zur Bereitstellung von Verbindungen der Formel (VII) erfolgen, wobei in den jeweiligen Verfahren Hydrazin an Stelle der Hydrazinverbindung der Formel R²HN-NH₂ verwendet wird.

Bezüglich der bevorzugten Reaktionsbedingungen bei der Umsetzung mit Hydrazin, gilt das zur Umsetzung von Verbindungen der Formel (V) mit Hydrazin Gesagte.

In einer alternativen Ausführungsform umfasst die Bereitstellung von Verbindungen der Formel (II) gemäß der vorliegenden Erfindung
A.1') die Bereitstellung einer Verbindung der Formel (VI), 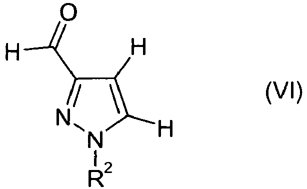 wobei R² eine der zuvor gegebenen Bedeutungen besitzt,
A.2') die Chlorierung oder Bromierung einer Verbindung der Formel (VI) in 4-Position des Pyrazols, wobei man eine Verbindung der Formel (X) erhält, 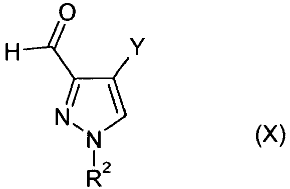 worin Y für Cl oder Br steht, sowie
A.3') die Überführung der Carbonylgruppe der Verbindung der Formel (X) in eine Dihalomethylgruppe umfasst.

Verbindungen der Formel (VI) können beispielsweise durch Umsetzung von 4-(Dimethylamino)-1,1-dimethoxy-3-en-2-on mit einer Hydrazinverbindung der Formel R²HN-NH₂ in Gegenwart einer geeigneten Base und anschließender saurer Aufarbeitung bereitgestellt werden. Die Umsetzung erfolgt üblicherweise in wässriger Lösung. Geeignete Basen sind beispielsweise Alkalimetallhydroxide, wie KOH oder NaOH. 4-(Dimethylamino)-1,1-dimethoxy-3-en-2-on lässt sich beispielsweise durch Umsetzung eines Dimethylformamidacetals mit Methylglyoxaldimethylacetal bereitstellen.

Die Bromierung der Verbindung der Formel (VI) erfolgt üblicherweise mit N-Bromsuccinimid (NBS) oder 1,3-Dibrom-5,5-dimethylhydantion (DDH). Die Chlorierung der Verbindung der Formel (VI) erfolgt entsprechend mit N-Chlorsuccinimid (NCS). Die Verwendung eines polaren Lösungsmittels, wie Dimethylformamid, N-Methylpyrrolidinon, 1,3-Dimethyl-2-imidazolidinon, Dimethylacetamid, Dimethylethylenharnstoff, Dimethylpropylenharnstoff (DMPU) oder Tetramethylharnstoff, hat sich für die Halogenierung von Verbindungen der Formel (VI) als besonders geeignet erwiesen. Die Reaktionstemperatur liegt üblicherweise in einem Bereich von -10 bis 80°C. Weitere Reaktionsbedingungen für die Halogenierung, die ebenfalls zielführend sind, sind dem Fachmann bekannt.

Für die Überführung der Aldehydgruppe der Verbindung (VI) in eine Dihalomethylgruppe, speziell in eine Difluormethylgruppe, hat sich beispielsweise die Umsetzung einer zuvor chlorierten oder bromierten Verbindung der Formel (VI) mit Dimethylaminoschwefeltrifluorid (DAST) als geeignet erwiesen. Diese Umsetzung wird üblicherweise in einem inerten Lösungsmittel, wie Dichlormethan, bei einer Temperatur im Bereich von -50 bis -10 °C, speziell bei einer Temperatur von etwa -20 °C, durchgeführt. DAST wird üblicherweise in einer Menge von 2 bis 4, bevorzugt 2,5 bis 3 Moläquivalenten, bezogen auf ein Mol der zuvor chlorierten oder bromierten Verbindung der Formel (VI) eingesetzt.

Alternativ kann die Carbonylgruppe durch Umsetzung mit Thionylchlorid in Gegenwart von Triphenylphosphin in eine Dichlormethylgruppe überführt werden. Diese kann gewünschtenfalls unter den zuvor für den Halogenaustausch beschriebenen Bedingungen in eine Difluormethylgruppe überführt werden.

In einer weiteren erfindungsgemäßen Ausführungsform umfasst die Bereitstellung von Verbindungen der Formel (II), worin Y für Cl steht, die Umsetzung von 1,2-Dichlorethen mit Dihaloacetylchlorid in Gegenwart einer Lewissäure, wie AlCl₃, zu (Z)-1,1-Dihalo-3,4-dihalobut-3-en-2-on, die Umsetzung des erhaltenen Reaktionsprodukts mit einer Hydrazinverbindung der Formel R²HN-NH₂, worin R² eine der zuvor gegebenen Bedeutungen besitzt, sowie die anschließende Überführung der Dichlormethylgruppe durch Halogenaustausch in eine Difluormethylgruppe. Bezüglich der speziellen Ausführungen der Umsetzung mit einer Hydrazinverbindung der Formel R²HN-NH₂ sowie zum Halogenaustausch wird auf die entsprechenden Stellen der Beschreibung verwiesen. Diese gelten sinngemäß.

Ein weiterer Gegenstand der vorliegenden Erfindung betrifft somit ein erfindungsgemäßes Verfahren, worin die Bereitstellung einer Verbindung der Formel (II), worin Y für Cl steht,
A.1") die Bereitstellung einer Verbindung der Formel (XI), 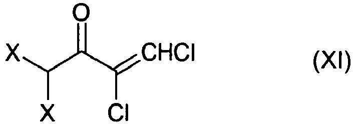 worin X für F oder Cl steht,
A.2") die Umsetzung einer Verbindung der Formel (XI) mit einer geeigneten Hydrazin-verbindung der Formel R²HN-NH₂ zu einer Verbindung der allgemeinen Formel (II), worin X für F oder Cl steht, Y für Cl steht und R² eine der zuvor gegebenen Bedeutungen besitzt, sowie
A.3") gegebenenfalls für den Fall, dass X in der Verbindung der Formel (II) für Chlor steht, einen Halogenaustausch gegen Fluor umfasst.

Im Folgenden wird die Herstellung Difluormethyl-substituierter Pyrazol-4-ylcarboxamide sowie deren Synthesevorstufen anhand von Beispielen erläutert.

### Beispiele

### 1. Herstellung von N-(2-(3,4,5-Trifluorphenyl)-3-difluormethyl-1-methylpyrazol-4-carbonsäureamid

### 1.1 Herstellung von 1,1-Difluor-4-ethoxy-3-buten-2-on (und 1,1-Difluor-4,4-diethoxy-2-butanon)

Zu Ethylvinylether (99%ig, 120 g, 1,375 mol) wurde bei 0°C Difluoracetylchlorid (41,4 g, 0,36 mol) aus einem auf -10°C gekühlten Tropftrichter innerhalb von 37 min getropft. Nach 7 h wurde auf 22°C erwärmt und weitere 12 h gerührt. Anschließend wurde das leicht viskose gelbliche Reaktionsgemisch (133 g) einer Flashdestillation unterworfen. Es wurden 48,5 g Destillat (Übergangstemp. 52 - 55°C bei 0,4 mbar) erhalten. Außerdem wurden weitere 30 g in der Kühlfalle einkondensiert. Das Destillat enthielt 1,1-Difluor-4-ethoxy-3-buten-2-on (54,7 %-GC-FI.) und 1,1-Difluor-4,4-diethoxy-2-butanon (30,9 %-GC-FI. 10,4 min). Das Kühlfallenkondensat enthielt 1,1-Difluor-4-ethoxy-3-buten-2-on (13,2 GC-FI.-%) und 1,1-Difluor-4,4-diethoxy-2-butanon (17,1 GC-FI.-%).
1,1-Difluor-4-ethoxy-3-buten-2-on:
EI-MS (GC-MS) [m/z]: 150 [M]⁺; ¹³C-NMR (125 MHz, CDCl₃): δ = 14,4, 68,4, 98,9, 110,7, 166,8, 187,4 ppm;
1,1-Difluor-4,4-diethoxy-2-butanon:
EI-MS [m/z] = 151; ¹³C-NMR (125 MHz, CDCl₃): δ = 15,2, 46,53, 62,55, 98,9, 110,7, 196,5 ppm.

### 1.2 Herstellung von 3-Difluormethyl-N-methylpyrazol

Zu einer Lösung von Methylhydrazin (99%ig, 11,9 g, 0,258 mol) in Essigsäure (460 ml) wurde bei 22°C eine Lösung von 45 g eines 85,6 %igen Gemischs, das 1,1-Difluor-4-ethoxy-3-buten-2-on (0,164 mol) und 1,1-Difluor-4,4-diethoxy-2-butanon (0,071 mol) im Verhältnis 2,3 / 1 enthielt, in Essigsäure (30 ml) über 37 min getropft. Die erhaltene Reaktionslösung wurde 19 h bei 22°C gerührt. Anschließend wurde die Essigsäure unter vermindertem Druck entfernt (43°C/30 mbar). Der ölige Rückstand wurde in Methyl-tert-Butylether (MTBE, 300 ml) aufgenommen und mit Wasser (250 ml) gewaschen. Die wässrige Phase wurde einmal mit MTBE (150 ml) extrahiert. Die gesammelten organischen Lösungen wurden über Natriumsulfat getrocknet, filtriert und unter vermindertem Druck vom Lösungsmittel befreit (40°C/400 bis 30 mbar). Der Rückstand (23,5 g) wurde zur Isolierung des Produkts fraktionierend destilliert. Die Hauptfraktion (21,5 g; Übergangstemperatur ≤ 52°C bei 22 mbar) enthielt 3-Difluor-N-methylpyrazol und 5-Difluor-N-methylpyrazol im Verhältnis 2,6 : 1 (GC-Retentionszeiten: 5-Isomer: 7,6 min; 3-Isomer: 9,2 min). Berücksichtigt man die in den Nebenfraktionen und Kühlfallen gefundenen Produktmengen, ergibt sich eine Ausbeute von 75,5% (Summe beider Isomere).
¹H-NMR (400 MHz, CDCl₃): δ = 3,95 (s, 3H), 6,42 (s, 1 H), 6,68 (t, 1H,), 7,4 ppm (s, 1H).

### 1.3 Herstellung von 4-Brom-3-difluormethyl-1-methylpyrazol

### 1.3.a) Halogenierung mit Brom

Zu einer Lösung von 3-Difluormethyl-N-methylpyrazol (2,0 g) in Methylenchlorid (80 ml) wurde bei 25 °C Brom (Br₂, 3 g) zugetropft. Das Reaktionsgemisch wurde insgesamt 22 h bei 25 °C gerührt und anschließend mit wässriger Natriumthiosulfatlösung (0,1 M, 210 ml) versetzt. Nach Phasentrennung wurde die organische Phase unter vermindertem Druck vom Lösungsmittel befreit (40 °C / 5 mbar). Ein Teil des erhaltenen Rückstandes (2,4 g von 2,8 g) wurde durch Säulenchromatographie (SiO₂, Ethylacetat/Cyclohexan 1:6) aufgetrennt. 4-Brom-3-difluormethyl-1-methylpyrazol wurde in einer Menge von 1,4 g isoliert.

EI-MS [m/z]: 210, 212 [M]⁺; ¹H-NMR (400 MHz, CDCl₃): δ = 3,9 (s, 3H), 6,68 (t, 1 H), 7,43 ppm (s, 1H); ¹³C-NMR (125 MHz, CDCl₃): δ = 39,84, 91,47, 110,48, 132,10, 143,54 ppm.

### 1.3.b) Halogenierung mit NBS

Zu einer Lösung von 3-Difluormethyl-N-methylpyrazol (5,0 g, 0,04 mol) in Dimethylformamid (DMF, 40 ml) wurde bei 3 - 5°C eine Lösung von N-Bromsuccinimid (NBS, 7,9 g, 0,04 mol) in DMF (20 ml) zugetropft (leicht exotherm). Das Reaktionsgemisch wurde anschließend auf Raumtemperatur erwärmt und weitere 2 h gerührt. Danach wurde das Reaktionsgemisch unter Rühren auf ein Gemisch aus Wasser (200 ml) und Natronlauge (konz., 5 ml) gegeben und viermal mit Methyl-tert-butylether (MTBE, 100 ml) extrahiert. Die vereinigten organischen Phasen wurden mit ges. wässriger NaCl-Lsg. gewaschen, über Magnesiumsulfat getrocknet, filtriert und anschließend unter vermindertem Druck vom Lösungsmittel befreit. Man erhielt 4-Brom-3-difluormethyl-N-methylpyrazol als Öl (9,5 g; Reinheit: 94,5 % (GC-Analyse); Ausbeute 95,2 %).

### 1.4 Herstellung von N-(2-(3,4,5-Trifluorphenyl)phenyl)-3-difluormethyl-1-methylpyrazol-4-carbonsäureamid

Zu einer Lösung von 4-Brom-3-difluormethyl-1-methylpyrazol (5,00 g, 23,7 mmol) in Acetonitril (100 ml) wurde im Autoklaven 2-(3,4,5-Trifluorphenyl)anilin (5,29 g, 23,7 mmol), Triethylamin (1,20 g, 11,8 mmol), Palladiumchlorid (86 mg, 0,47 mmol), wasserfreies Kaliumcarbonat (6,55 g, 47,4 mmol) und 2,2-Dimethyl-1,3-bis(diphenylphosphino)propan (313 mg, 0,71 mmol) gegeben. Der Autoklav wurde dreimal mit 9 bar Stickstoff und anschließend 3 mal mit 10 bar Kohlenmonoxid gespült. Das Reaktionsgemisch wurde unter einem Kohlenmonoxid-Druck von 9 mbar bei einer Außentemperatur von 130°C 20 h gerührt. Die nach Abkühlen und Entspannen erhaltene rotbraune Suspension wurde filtriert und das Filtrat unter vermindertem Druck von flüchtigen Bestandteilen befreit. Der Rückstand wurde in einer Mischung aus Tetrahydrofuran (60 ml) und Methyl-tert-butylether (MTBE, 60 ml) aufgenommen, zweimal mit Salzsäure (5%ig, je 40 ml) und einmal mit Wasser (30 ml) gewaschen, über Natriumsulfat getrocknet, filtriert und unter vermindertem Druck vom Lösungsmittel befreit. Der Rückstand wurde mit Diisopropylether (20 ml) verrührt, der Feststoff wurde abfiltriert und am Vakuum getrocknet. 5,20 g (Ausbeute 54 %) N-(2-(3,4,5-Trifluorphenyl)-phenyl)-3-difluormethyl-1-methylpyrazol-4-carbonsäureamid wurde laut HPLC-Analyse (Merck Chromolith SpeedROD RP-18e-Säule, 504,6 mm; Gradient: Acetonitril/Wasser mit 0,1 % Trifluoressigsäure, 7 min von 10 % auf 95 % Acetonitril) in einer Reinheit von 93 % isoliert.
¹H-NMR (CDCl₃): δ = 3,93 (s), 6,65 (t), 7,00 (m), 7,23 (m), 7,42 (m), 7.80 (s, br.), 7,96 (s), 8,20 ppm (m).

### 2. Palladiumkatalysierte Herstellung von N-(2-(3,4,5-Trifluorphenyl)-phenyl)-3-difluormethyl-1-methylpyrazol-4-carbonsäureamid mit verschiedenen PhosphinLiganden

Die Reaktionen wurden in einem Parallelreaktor (Chemspeed Accelerator) durchgeführt. Die Edukte wurden unter Stickstoffatmosphäre eingewogen oder als Lösung zugegeben. Der CO-Partialdruck betrug bei Raumtemperatur ~10 bar. Die Reaktorblöcke wurden unter Schütteln auf 130 - 150 °C erhitzt. Dabei stieg der Druck auf ~15 bar. Nach 16 h Reaktionszeit, Abkühlen und Entspannen des Reaktionsgemisches wurde Decan oder Diethylenglykoldiethylether (DEGEE) als interner Standard zugegeben und die Reaktionen anhand der GC-Flächen% der Hauptkomponenten ausgewertet.

### 2.a) Reaktion in Gegenwart von 1,3-bis(Dicyclohexylphosphino)propan (DCPP)

Unter Schutzgas wurden Pd(C₆H₄CN)₂Cl₂ (2,3 mg, 0,0060 mmol), DCPP (7,9 mg, 0,018 mmol), Diazabicycloundecen (DBU, 274,0 mg, 1,8 mmol), 4-Brom-3-difluormethyl-1-methylpyrazol (253,3 mg, 1,2 mmol) und 2-(3,4,5-Trifluorphenyl)anilin (267,8 mg, 1,2 mmol) eingewogen. Unter Argon wurde Dimethylformamid (DMF, 5 ml) zugegeben. Das Reaktionsgemisch wurde für 10 min geschüttelt. Anschließend wurde das Reaktionsgemisch bei einem CO-Druck von 15 bar bei einer Temperatur 130 °C für 16 h stark geschüttelt. Nach Abkühlen des Reaktionsgemisches wurde für die GC-Analyse DEGEE (0.2 ml) als interner Standard zugesetzt.

Ergebnis der GC-Analyse [Flächen-%]: 58,36 DMF; 4,14 DEGEE; 1,16 4-Brom-3-difluormethyl-1-methylpyrazol; 0,11 2-(3,4,5-Trifluorphenyl)anilin; 12,92 N-(2-(3,4,5-Trifluorphenyl)-phenyl)-3-difluormethyl-1-methylpyrazol-4-carbonsäureamid.

Dies entspricht einer formalen Ausbeute von > 90 %.

### 2.b) Reaktion in Gegenwart von 1,1'-bis(Diphenylphosphino)ferrocen (DPPF)

Unter Schutzgas wurden Pd(C₆H₄CN)₂Cl₂ (2,3 mg, 0,0060 mmol), DPPF (10,0 mg, 0,018 mmol), Triethylamin (91,1 mg, 0,9 mmol), Kaliumcarbonat (124,4 mg, 0,9 mmol), 4-Brom-3-difluormethyl-1-methylpyrazol (253,3 mg, 1,2 mmol) und 2-(3,4,5-Trifluorphenyl)anilin (267,8 mg, 1,2 mmol) eingewogen. Unter Argon wurde Acetonitril (5 ml) zugegeben und das erhaltene Reaktionsgemisch für 10 min geschüttelt. Anschließend wurde das Reaktionsgemisch bei einem CO-Druck von 15 bar bei einer Temperatur von 130 °C für 16h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 76,49 Acetonitril; 3,87 DEGEE; 0,00 4-Brom-3-difluormethyl-1-methylpyrazol; 2,10 2-(3,4,5-Trifluorphenyl)anilin; 12,25 N-(2-(3,4,5-Trifluorphenyl)-phenyl)-3-difluormethyl-1-methylpyrazol-4-carbonsäureamid.

Dies entspricht einer formalen Ausbeute von > 85 %.

### 2.c) Reaktion in Gegenwart von 1,3-bis(Di-(2,6-dimethoxyphenyl)phosphino)propan ((o-MeO)-DPPP)

Unter Schutzgas wurden Pd(C₆H₄CN)₂Cl₂ (23 mg, 0,0060 mmol), (o-MeO)-DPPP (9,6 mg, 0,018 mmol), Kaliumcarbonat (248,8 mg, 1,8 mmol), 4-Brom-3-difluormethyl-1-methylpyrazol (253,3 mg, 1,2 mmol) und 2-(3,4,5-Trifluorphenyl)anilin (26,8 mg, 1,2 mmol) eingewogen. Unter Argon wurde N-Methylpyrrolidon (NMP, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 83,14 NMP; 1,25 DEGEE; 0,17 4-Brom-3-difluormethyl-1-methylpyrazol; 3,15 2-(3,4,5-Trifluorphenyl)anilin; 2,87 N-(2-(3,4,5-Trifluorphenyl)-phenyl)-3-difluormethyl-1-methylpyrazol-4-carbonsäureamid.

Dies entspricht einer formalen Ausbeute von > 34 %.

### 2.d) Reaktion in Gegenwart von 1,3-bis(Dicyclohexylphosphino)propan (DCPP)

Unter Schutzgas wurden Pd(C₆H₄CN)₂Cl₂ (2,3 mg, 0,0060 mmol), DCCP (7,9 mg, 0,018 mmol), Triethylamin (91,1 mg, 0,9 mmol), Kaliumcarbonat (124,4 mg, 0,9 mmol), 4-Brom-3-difluormethyl-1-methylpyrazol (253.3 mg, 1,2 mmol) und 2-(3,4,5-Trifluorphenyl)anilin (26,8 mg, 1,2 mmol) eingewogen. Unter Argon wurde Acetonitril (MeCN, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 76,10 MeCN; 3,86 DEGEE; 1,20 4-Brom-3-difluormethyl-1-methylpyrazol; 4,26 2-(3,4,5-Trifluorphenyl)anilin; 8,54 N-(2-(3,4,5-Trifluorphenyl)-phenyl)-3-difluormethyl-1-methylpyrazol-4-carbonsäureamid.

Dies entspricht einer formalen Ausbeute von > 61 %.

### 2.e) Reaktion in Gegenwart von 2-Butyl-2-ethyl-1,3-bis-(diphenylphosphino)-propan (Bustar, Et,Bu-Pepstar)

Unter Schutzgas wurden Pd(C₆H₄CN)₂Cl₂ (2,3 mg, 0.0060 mmol), Bustar (8,9 mg, 0,018 mmol), Triethylamin (91,1 mg, 0,9 mmol), Kaliumcarbonat (124,4 mg, 0,9 mmol), 4-Brom-3-difluormethyl-1-methylpyrazol (253,3 mg, 1,2 mmol) und 2-(3,4,5-Trifluorphenyl)anilin (26,8 mg, 1,2 mmol) eingewogen. Unter Argon wurde Acetonitril (MeCN, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 77,50 MeCN; 3,88 DEGEE; 0.04 4-Brom-3-difluormethyl-1-methylpyrazol; 3,23 2-(3,4,5-Trifluorphenyl)anilin; 9,89 N-(2-(3,4,5-Trifluorphenyl)-phenyl)-3-difluormethyl-1-methylpyrazol-4-carbonsäureamid.

Dies entspricht einer formalen Ausbeute von > 70 %

### 2.f) Reaktion in Gegenwart von 1,3-bis(Di-(2,6-dimethoxyphenyl)phosphino)propan (o-MeO-DPPP)

Unter Schutzgas wurden Pd(C₆H₄CN)₂Cl₂ (2,3 mg, 0,0060 mmol), o-MeO-dppp (9,6 mg, 0,018 mmol), Kaliumphosphat (382,1 mg, 1,8 mmol), 4-Brom-3-difluormethyl-1-methylpyrazol (25,3 mg, 1,2 mmol) und 2-(3,4,5-Trifluorphenyl)anilin (26,8 mg, 1,2 mmol) eingewogen. Unter Argon wurde Dimethylacetamid (DMA, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16 h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 80,12 DMA; 3,74 DEGEE; 1,50 4-Brom-3-difluormethyl-1-methylpyrazol; 3,34 2-(3,4,5-Trifluorphenyl)anilin; 8,00 N-(2-(3,4,5-Trifluorphenyl)-3-difluormethyl-1-methylpyrazol-4-carbonsäureamid.

Dies entspricht einer formalen Ausbeute von > 62 %

### 2.g) Reaktion in Gegenwart von 2,2-Dimethyl-1,3-bis-(diphenylphosphino)propan (Pepstar)

Unter Schutzgas wurden Pd(C₆H₄CN)₂Cl₂ (2,3 mg, 0,0060 mmol), Pepstar (7,9 mg, 0,018 mmol), Kaliumcarbonate (248,8 mg, 1,8 mmol), 4-Brom-3-difluormethyl-1-methylpyrazol (253,3 mg, 1,2 mmol) und 2-(3,4,5-Trifluorphenyl)anilin (26,8 mg, 1,2 mmol) eingewogen. Unter Argon wurde N-Methylpyrrolidon (NMP, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16 h stark geschüttelt. Nach Abkühlen des Reaktionsgemisches wurde für die GC-Analyse DEGEE (0.2 ml) als interner Standard zugesetzt.

Ergebnis der GC-Analyse [Flächen-%]: 17,98 NMP; 6,13 DEGEE; 2,11 4-Brom-3-difluormethyl-1-methylpyrazol; 3,76 2-(3,4,5-Trifluorphenyl)anilin; 13,48 N-(2-(3,4,5-Trifluorphenyl)phenyl)-3-difluormethyl-1-methylpyrazol-4-carbonsäureamid.

### Dies entspricht einer formalen Ausbeute von > 70 %

### 2.h) Reaktion in Gegenwart von 1-[2-(Bis(ethyl)phosphino)ferrocenyl]ethyl-di-tert-butylphosphin (Josiphos tBu₂P-Fc-PEt₂)

Unter Schutzgas wurden Pd(C₆H₄CN)₂Cl₂ (2,3 mg, 0,0060 mmol), Josiphos tBu₂P-Fc-PEt₂ (10,4 mg; 0,018 mmol), DBU (274,0 mg, 1,8 mmol), 4-Brom-3-difluormethyl-1-methylpyrazol (253,3 mg, 1,2 mmol) und 2-(3,4,5-Trifluorphenyl)anilin (26,8 mg, 1,2 mmol) eingewogen. Unter Argon wurde Dimethylformamid (DMF, 5 ml) zugegeben. Das Reaktionsgemisch wurde für 10 min geschüttelt. Anschließend wurde das Reaktionsgemisch bei einem CO-Druck von 15 bar bei einer Temperatur von 130 °C für 16 h stark geschüttelt. Nach Abkühlen des Reaktionsgemisches wurde für die GC-Analyse DEGEE (0,2 ml) als interner Standard zugesetzt.

Ergebnis der GC-Analyse [Flächen-%]: 58,85 DMF; 4,48 DEGEE; 0,37 4-Brom-3-difluormethyl-1-methylpyrazol; 4,34 2-(3,4,5-Trifluorphenyl)anilin; 9,66 N-(2-(3,4,5-Trifluorphenyl)-3-difluormethyl-1-methylpyrazol-4-carbonsäureamid.

Dies entspricht einer formalen Ausbeute von > 67 %.

### 2.i) Untersuchung zur CO-Druckabhängigkeit der Reaktion in Gegenwart von 2,2-Dimethyl-1,3-bis-(diphenylphosphino)propan (Pepstar)

Eine Lösung von Pd(PhCN)₂Cl₂ (0.0375 mmol) und Pepstar (0.1125 mmol) in Dimethylformamid (DMF, 5 ml) wurde 30 Minuten bei Raumtemperatur gerührt. Die Lösung enthaltend den präformierten Katalysator wurde in einen inertisierten Autoklaven überführt. Eine Lösung von Diazabicycloundecen (DBU, 16.5 mmol), 3-Difluormethyl-1-methyl-4-brompyrazol (15 mmol) und 2-(3,4,5-Trifluorphenyl)anilin (15 mmol) in DMF (40 ml) wurde im CO-Gegenstrom zu der Lösung des Katalysators in den Autoklaven überführt. Die Reaktion wurde unter Rühren bei 150 °C und dem in Tabelle 1 angegebenen CO-Druck bis zur vollständigen Umsetzung des Brompyrazols durchgeführt.

**Tabelle 1: Versuche zur Carbonylierung bei verschiedenem CO-Druck**

| Versuch | CO-Druck [bar] | Reaktionszeit [h] | Isolierte Ausbeute [%] |
|---|---|---|---|
| 2.i.1 | 10 | 20 | 81 |
| 2.i.2 | 15 | 20 | 68 |
| 2.i.3 | 25 | 18.5 | 70 |
| 2.i.4 | 30 | 20 | 66 |
| 2.i.5 | 40 | 32 | 21 |

Nach beendeter Reaktion wurde das Reaktionsgemisch mit Methyl-tert-butylether (MTBE, 50 ml) versetzt und mit Wasser (30 ml) gewaschen. Die wäßrige Phase wurde mit MTBE (4 x 30 ml) extrahiert. Anschließend wurden die organischen Phasen vereinigt, getrocknet und unter vermindertem Druck vom Lösungsmittel befreit.

Der Rückstand wurde in Toluol (10 ml) gelöst und unter Rühren erwärmt. Anschließend wurde n-Hexan (10 ml) zugegeben. Nach wenigen Minuten bildeten sich Kristalle, die nach 1 h abfiltriert, mit Hexan gewaschen und getrocknet wurden. Das Filtrat wurde eingeengt, der dabei entstandene Rückstand in Toluol gelöst und mit n-Hexan versetzt. Weiteres Produkt konnte so aus dem Filtrat isoliert werden.

Die für die vollständige Umsetzung benötigte Reaktionszeiten sowie die bei der Reaktion isolierten Ausbeuten sind der Tabelle 1 zu entnehmen.

¹H-NMR (499.8 MHz, CDCl₃, interner Standard: TMS): d = 3.87 (s, 3 H), 6.71 (t, J = 54.2 Hz, 1 H); 6.98 (m, 2 H), 7.25 (m, 2 H), 7.39 (m, 1 H), 7.91 (br s, 2 H), 8.08 ppm (d, J = 8.2, 1 H).

¹³C-NMR (125.7 MHz, CDCl₃, TMS als interner Standard): d = 39.51 (s), 111.5 (t, J = 233.2 Hz), 113.60 (ddd, J = 16.2 Hz, J = 5.32 Hz), 116.52 (s), 123.79 (s), 125.41 (s), 129.22 (s), 130.11 (s), 131.58 (td, J = J = 1.0 Hz), 134.29 (td, J = 7.0 Hz, J = 5.0 Hz), 134.51 (s), 135.9 (s), 135.85 (s), 139.53 (dt, J = 252.4 Hz, J = 15.1 Hz), 142.7 (t, J = 28.9 Hz), 151.3 (ddd, J = 251.0 Hz, J = 9.9 Hz, J = 4.1 Hz), 159.65 ppm (s).

### 2.k) Reaktion in Gegenwart von 2,2-Dimethyl-1,3-bis-(diphenylphosphino)propan (Pepstar)

Eine Lösung von Pd(PhCN)₂Cl₂ (0.008 mmol) und Pepstar (0.024 mmol) in Dimethylformamid (DMF, 5 ml) wurde 30 Minuten bei Raumtemperatur gerührt. Die Lösung enthaltend den präformierten Katalysator wurde in einen inertisierten Autoklaven überführt. Diazabicycloundecen (DBU, 8.8 mmol), Tetrabutylammoniumbromid (0.32 mmol) sowie Lösungen von 3-Difluormethyl-1-methyl-4-brompyrazol (8.8 mmol) und 2-(3,4,5-Trifluorphenyl)anilin (8 mmol) in DMF (je 20 ml) wurden im CO-Gegenstrom zu der Lösung des Katalysators in den Autoklaven überführt. Das Reaktionsgemisch wurde 24 h bei 150 °C und bei einem CO-Druck von 15 bar gerührt.
Der Umsatz bezogen auf 2-(3,4,5-Trifluorphenyl)anilin betrug 76 % bei einer Selektivität von 100 %. Die Zusammensetzung des Reaktionsaustrags laut GC-Analyse war wie folgt: DMF 78.65 FI.-%, 3-Difluormethyl-1-methyl-4-b rompyrazol 0.0 FI.-%, DBU 12.98 FI.-%, 2-(3,4,5-Trifluorphenyl)anilin 1.99 [FI.-%] und N-(2-(3,4,5-Trifluorphenyl)-phenyl)-3-difluormethyl-1-methylpyrazol-4-carbon-säureamid 6.38 FI.-%.

Die Aufarbeitung erfolgte wie in Beispiel 2.i) beschrieben und ergab eine isolierte Ausbeute an N-(2-(3,4,5-Trifluorphenyl)-phenyl)-3-difluormethyl-1-methylpyrazol-4-carbonsäureamid von 45.4 %.

### 2.I) Reaktion in Gegenwart von 2-Butyl-2-ethyl-1,3-bis-(diphenylphosphino)-propan (Bustar)

Eine Lösung von Pd(PhCN)₂Cl₂ (0.0375 mmol) und Bustar (0.1125 mmol) in Dimethylformamid (DMF, 10 ml) wurde 30 Minuten bei Raumtemperatur gerührt. Die Lösung enthaltend den präformierten Katalysator wurde in einen inertisierten Autoklaven überführt. Diazabicycloundecen (DBU, 16.5 mmol), sowie Lösungen von 3-Difluormethyl-1-methyl-4-brompyrazol (16.5 mmol) und 2-(3,4,5-Trifluorphenyl)anilin (15 mmol) in DMF (je 25 ml) wurden im CO-Gegenstrom zu der Lösung des Katalysators in den Autoklaven überführt. Das Reaktionsgemisch wurde 24 h bei 150 °C und bei einem CO-Druck von 15 bar gerührt.
Der Umsatz bezogen auf 2-(3,4,5-Trifluorphenyl)anilin betrug 99 % bei einer Selektivität von 94 %. Die Zusammensetzung des Reaktionsaustrags laut GC-Analyse war wie folgt: DMF 44.6 FI.-%, Brompyrazol 0.1 FI.-%, DBU 29.7 FI.-%, 2-(3,4,5-Trifluorphenyl)anilin 0.3 [FI.-%] und N-(2-(3,4,5-Trifluorphenyl)-phenyl)-3-difluormethyl-1-methylpyrazol-4-carbonsäureamid 22.7 FI.-%.

Die Aufarbeitung erfolgte wie in Beispiel 2.i) beschrieben und ergab eine isolierte Ausbeute an N-(2-(3,4,5-Trifluorphenyl)-phenyl)-3-difluormethyl-1-methylpyrazol-4-carbonsäureamid von 78 %.

### 3. Herstellung von 3-Difluormethyl-N-methylpyrazol durch Umsetzung von 3-Difluormethyl-NH-pyrazol mit Hydrazin und anschließender N-Methylierung

### 3.1 Herstellung von 3-Difluormethyl-NH-pyrazol

Zu einer Lösung von Hydrazin-Hydrat (100%ig, 0,31 g, 0,063 mol) in Essigsäure (10 ml) wurde bei einer Temperatur von 22 bis 31 °C eine Lösung von 1 g eines 86%igen Gemisches, das 1,1-Difluor-4-ethoxy-3-buten-2-on und 1,1-Difluor-4,4-di-ethoxy-2-butanon im Verhältnis 1,5 : 1 enthielt, in Essigsäure (3 ml) über 26 min getropft. Die Lösung wurde 64 h bei 22°C gerührt. Anschließend wurde die Essigsäure abdestilliert (42°C/30 mbar). Der ölige Rückstand wurde in Methyl-tert-Butylether (MTBE, 4 ml) aufgenommen und dreimal mit Wasser (10 ml) gewaschen. Eine Probe der MTBE-Lösung wurde unter vermindertem Druck aufkonzentriert (39°C/ 25 mbar, später 0,4 mbar).
EI-MS (GC-MS) [m/z]: 118 [M]⁺; ¹H-NMR (400 MHz, CDCl₃): δ = 6,65 (s, 1 H),
6,8 (t, 1 H), 7,68 (s, 1 H), 10,2 ppm (s, breit, NH); ¹³C-NMR (125 MHz, CDCl₃): δ = 103,41, 111,09, 130,22, 146,41 ppm.

### 3.2 Herstellung von 3-Difluormethyl-N-methylpyrazol

a) Zu einer Lösung von Hydrazin-Hydrat (14,7 g, 0,294 mol) in Essigsäure (600 ml) wurde bei 22°C eine Essigsäurelösung (40 ml) von 59 g eines 77,4 %igen Gemischs, das 1,1-Difluor-4-ethoxy-3-buten-2-on (0,145 mol) und 1,1-Difluor-4,4-diethoxy-2-butanon (0,122 mol) im Verhältnis 1,1 / 1 enthielt, über 76 min getropft. Die Lösung wurde 16 h bei 22°C gerührt. Anschließend wurde die Essigsäure unter vermindertem Druck entfernt (45°C/28 mbar). Der ölige Rückstand wurde in Essigester (450 ml) aufgenommen und mit Wasser (450 ml) gewaschen. Die wässrige Phase wurde einmal mit Essigester (250 ml) re-extrahiert. Die trübe Wasserphase wurde über Kieselgur filtriert. Das Kieselgur wurde mit wenig Essigester nachgewaschen, die Wasserphase nochmals mit MTBE (250 ml) re-extrahiert. Die gesammelten organischen Lösungen wurden über Natriumsulfat getrocknet, filtriert und unter vermindertem Druck von flüchtigen Bestandteilen befreit. Es verblieb ein Rückstand von 41,5 g.
b) Ein Teil des Rückstands (37,4 g, 0,24 mol) wurde in Aceton (540 ml) gelöst. Zu der Lösung wurde Kaliumcarbonat (57,4 g) gegeben. Anschließend wurde Dimethylsulfat (52,4 g, 0,42 mol) über 73 min zugetropft. Dabei erwärmte sich das Reaktionsgemisch auf 31°C. Anschließend wurde das Reaktionsgemisch bei einer Temperatur von 28,5 bis 30°C für 105 min, weitere 18 h bei 21°C und weitere 75 min bei 29 bis 37°C gerührt. Das Reaktionsgemisch enthielt 2,7% (GC-FI.) Edukt sowie 6,8 % (GC-FI.) Dimethylsulfat. Anschließend wurden am Rotationsverdampfer ca. 390 g Aceton entfernt (40°C / 300 mbar). Der Rückstand wurde in Wasser (300 ml) und Essigester (200 ml) aufgenommen. Nach Phasentrennung wurde die wässrige Phase mit Essigester (100 ml) extrahiert. Die gesammelten organischen Phasen wurden über Natriumsulfat (30 g) getrocknet, filtriert und unter vermindertem Druck vom Lösungsmittel befreit (40°C / 400 bis 300 mbar). Der Rückstand (40,6 g) wird zur Isolierung des Produkts fraktionierend destilliert. Die Hauptfraktion (20,4 g, Übergangstemp. 20 - 65°C bei 20 mbar) enthielt 3-Difluor-N-methylpyrazol und 5-Difluor-N-methylpyrazol im Verhältnis 4,7 : 1 (GC-Retentionszeiten: 5-Isomer: 7,6 min; 3-Isomer: 9,2 min).
   Ausbeute über Stufe a) und b) (nach Destillation): 47 % (Summe beider Isomere). Berücksichtigt man die in den Nebenfraktionen und Kühlfallen gefundenen Produktmengen, ergibt sich eine korrigierte Ausbeute von 58%. 3-Difluormethyl-N-methylpyrazol:
   ¹H-NMR (400 MHz, CDCl₃): δ = 3,95 (s, 3H), 6,42 (s, 1 H), 6,68 (t, 1 H), 7,4 ppm (s, 1 H); 5-Difluormethyl-N-methylpyrazol:
   ¹H-NMR (400 MHz, CDCl₃): δ = 4,95 (s, 3H), 6,42 (s, 1 H), 6,68 (t, 1 H), 7,4 ppm (s, 1 H).

Das so erhaltene 3-Difluormethyl-N-methylpyrazol kann in Beispiel 1 eingesetzt werden.

### 4. Herstellung von 3-Dichlormethyl-N-methylpyrazol via in situ Herstellung von 1,1-Dichlor-4-ethoxy-3-buten-2-on

Dichloracetylchlorid (98 %ig, 30,2 g, 0,2 mol) wurde unter Rückflussbedingungen (38 °C) zu Ethylvinylether (200 ml, ca. 2,09 mol) gegeben. Nach 3 h wurden etwa 60 ml des überschüssigen Ethylvinylethers bei 50°C abdestilliert. Anschließend wurde Toluol (100 ml) zugegeben. Flüchtige Bestandteile wurden zusammen mit Toluol unter vermindertem Druck (50°C / 60 mbar) entfernt. Der Rückstand wurde erneut mit Toluol (100 ml) versetzt. Die so hergestellte gelbliche Lösung wurde auf Rückflusstemperatur erhitzt. Anschließend wurde bei gleichzeitigem Abdestillieren des Toluols eine Lösung von Methylhydrazin (98%ig, 9,4 g, 0,2 mol) in Toluol (100 ml) über einen Zeitraum von 70 min zugetropft. Zeitgleich wurde Toluol in einer Menge entsprechend dem als Destillat erhaltene Volumen dem Reaktionsgemisch kontinuierlich zugeführt. Nach weiteren 30 min Rühren wurde auf 25°C abgekühlt und mit Wasser (1x100 ml und 1x50 ml) gewaschen. Die wässrigen Phasen werden mit Methylenchlorid (2x100 ml) extrahiert. Die organischen Phasen wurden vereinigt und unter vermindertem Druck vom Lösungsmittel befreit (50°C/15 mbar). Der Rückstand (25,3 g) wurde durch Flashdestillation aufgetrennt. Als Destillat (12,9 g, Übergangstemp. 56-60°C / 0,3 bis 0,2 mbar) wurde ein Isomerengemisch aus 3-Dichlormethyl-N-methylpyrazol und 5-Dichlormethyl-N-methylpyrazol im Verhältnis 80 : 20 (GC-FI. %) erhalten. Dies entspricht einer Ausbeute an 3-Dichlormethyl-N-methylpyrazol von 36 %.

### 4a. Herstellung von 1,1-Dichlor-4-ethoxy-3-buten-2-on

Dichloracetylchlorid (95 %ig, 16,5 g, 0,11 mol) und Ethylvinylether (99 %ig, 48,7 g, 0,67 mol) wurden bei ca. 0°C zusammengegeben und 5 h gerührt. Anschießend wurde etwa 11 h bei 22°C weiter gerührt. Anschließend wurde der Großteil des überschüssigen Ethylvinylethers unter vermindertem Druck destillativ entfernt (40°C / 300-400 mbar). Aus dem Rückstand (18,7 g) wurde das Produkt durch Flashdestillation (Ölbad 100°C / 0,7 mbar; Übergang 72°C) abgetrennt. Als Destillat wurde ein Produktgemisch (15,6 g) erhalten. Dieses bestand zu 80 % (GC-FI.-%) aus dem gewünschten Produkt. Weiterhin bestand das Produktgemisch zu etwa 5 % (GC-FI.%) aus 1,1-Dichlor-4,4-diethoxy-2-butanon. Die Ausbeute betrug inklusive dem entsprechenden Diethylacetal etwa 68%.

Das so erhaltene 1,1-Dichlor-4-ethoxy-3-buten-2-on kann beispielsweise analog zu Beispiel 3 durch Umsetzung mit Methylhydrazin in 3-Dichlormethyl-N-methylpyrazol überführt werden.

### 5. Herstellung von 3-Dichlormethyl-N-methylpyrazol ausgehend von 1,1,4-Trichlor-3-buten-2-on

Zu einer Lösung von 1,1-Dimethylhydrazin (9,5 g, 0,156 mol) in Methyl-tert-butylether (MTBE, 500 ml) wurde bei 20°C 1,1,4-Trichlor-3-buten-2-on (15 g, 0,078 mol) unter Erwärmung auf 34°C in 37 min zugetropft. Nach 2 h hatte sich eine klebrig braune Suspension gebildet, die über Kieselgur filtriert wird. Das Filtrat wurde unter vermindertem Druck eingeengt. Der Rückstand (7,6 g eines viskosen Öls) wurde einer fraktionierenden Destillation unter vermindertem Druck unterworfen. Als Hauptfraktion wurden 3 g 3-Dichlormethyl-N-methylpyrazol in einer Reinheit von 96,4% (GC-FI.) erhalten (Siedetemp. 65°C/ 0,3 mbar), die beim Stehen lassen kristallisiert. Durch Umkristallisation wurde eine Probe mit einer Reinheit > 99 % (GC-FI.) erhalten. Smp.: 38,5-40°C; ¹H-NMR (400 MHz, CDCl₃): δ = 3,88 (s, 3H), 6,5 (s, 1 H), 6,8 (s, 1 H), 7,34 ppm (s, 1H); ¹³C-NMR (125 MHz, CDCl₃): δ=65,24, 104,10, 131,60, 151,50 ppm.

### 6. Herstellung von 3-Dichlormethyl-N-methylpyrazol via in situ Herstellung von 1,1-Dichlor-4-ethoxy-3-buten-2-on und Umsetzung mit Methylhydrazin

Dichloracetylchlorid (98 %ig, 15,1 g, 0,1 mol) wurde bei 10°C zu Ethylvinylether (100 ml, ca. 1,04 mol) gegeben. Nach 16 h wurden ca. 30 ml des überschüssigen Ethylvinylethers bei 50°C abdestilliert. Danach wurde Toluol (100 ml) zugegeben und flüchtige Bestandteile mit Toluol unter vermindertem Druck (50°C / 60 mbar) coevaporiert. Der Rückstand wurde in Toluol (etwa 100 ml) gelöst. Diese Lösung wurde mit Essigsäure (100 ml) versetzt. Anschließend wurde unter Erwärmung auf 30°C eine wässrigen Methylhydrazin-Lösung (40 %ig,11,5 g, 0,1 mol) über 15 min zugetropft. Es bildete sich ein 2-phasiges Gemisch, das weitere 12 h bei 25°C gerührt wurde. Anschließend wurden die Phasen getrennt und das Toluol unter vermindertem Druck abdestilliert (50°C/ 10 mbar). Der Rückstand wurde in Toluol (100 ml) aufgenommen und erneut aufkonzentriert. Als Rückstand wurden 12,0 g eines Isomerengemischs von 3-Dichlormethyl-N-methylpyrazol und 5-Dichlormethyl-N-methylpyrazol im Verhältnis von 57 : 43 (GC-FI.-%) erhalten.

### 7. Herstellung von 3-Dichlormethyl-NH-pyrazol ausgehend von 1,1-Dichlor-4-ethoxy-3-buten-2-on

Zu einer Lösung von Hydrazin-Hydrat (9,7 g, 0,193 mol) in Essigsäure (100 ml) wurde bei 22°C eine Lösung von 36,4 g eines Gemischs, enthaltend 1,1-Dichlor-4-ethoxy-3-buten-2-on (0,154 mol) und 1,1-Dichlor-4,4-diethoxy-2-butanon (21,5 mmol), in Essigsäure (20 ml) über 50 min zugetropft. Die Lösung wurde für 20 h bei 22°C gerührt. Anschließend wurde die Essigsäure destillativ entfernt (42°C/25 mbar, später 0,2 mbar). Das ölige Rückstand wurde in Methyl-tert-butylether (MTBE, 150 ml) aufgenommen und dreimal mit Wasser (30 ml) gewaschen. Die organische Phase wurde über Kieselgur filtriert. Das Filtrat wurde aufkonzentriert (39°C/ 400 bis 25 mbar, später 0,4 mbar).

Es wurden 27 g eines Rohprodukts erhalten, das noch ca. 9 % Essigsäure enthielt. Dieser wurde ohne weitere Aufreinigung in den Folgereaktionen eingesetzt.
EI-MS (GC-MS) [m/z]: 150 [M]⁺; ¹H-NMR (400 MHz, CDCl₃): δ = 6,65 (s, 1H), 6,9
(s, 1 H), 7,75 (s, 1 H), 10,5 ppm (s, breit, 1H); ¹³C-NMR (125 MHz, CDCl₃): δ = 66,16, 102,76, 130,35, 150,31 ppm.

### 8. Herstellung von 3-Difluorchlormethyl-NH-pyrazol ausgehend von 4-Butoxy-1-chloro-1,1-difluoro-but-3-en-2-on

Zu einer Lösung von 4-Butoxy-1-chloro-1,1-difluoro-but-3-en-2-on (1,2 g, 0,005 mol) in Ethanol (6 ml) wurde innerhalb von 10 Minuten Hydrazin-Hydrat (80 %ig, 0,52 g, 0,008 mol) getropft. Anschließend wurde 5 h unter Rückflussbedingungen gerührt. Die erhaltene gelb-braunen Suspension wurde am Rotationsverdampfer eingeengt. Der Rückstand wurde mit Essigester (20 ml) und Wasser (20 ml) aufgenommen. Nach Trennung der Phasen wurde die wässrige Phase erneut mit Essigester (20 ml) extrahiert. Die organischen Phasen wurden vereint, über MgSO₄ getrocknet, filtriert und unter vermindertem Druck von flüchtigen Bestandteilen befreit. Der hellrote ölige Rückstand (0,35 g) bestand etwa zu 65 % aus 3-Difluorchlormethyl-NH-pyrazol und zu 35 % aus Pyrazol-3-carbonsäureethylester.
3-Difluorchlormethyl-NH-pyrazol: EI-MS [m/z]: 152 [M]⁺; ¹H-NMR (500 MHz, CDCl₃):
δ = 6,68 (s, 1 H), 7,9 (s, 1H), 11,5-13,5 ppm (s, breit, NH); ¹³C-NMR (125 MHz, CDCl₃):
δ = 103,61, 124,76, 131,18, 147,91 ppm.
Pyrazol-3-carbonsäureethylester: EI-MS [m/z]: 140 [M]⁺; ¹H-NMR (500 MHz, CDCl₃):
δ = 1,38 (t, 3H), 4,32 (q, 2H), 6,8 (s, 1 H), 7,8 (s, 1 H), 11,5-13,5 ppm (s, breit, NH); ¹³C-NMR (125 MHz, CDCl₃): δ = 14,24, 60,2, 107,80, 132,30, 141,00, 161,60 ppm.

### 9. Herstellung von 3-Difluorchlormethyl-N-methylpyrazol ausgehend von 4-Butoxy-1-chlor-1,1-difluorbut-3-en-2-on

Zu einer Lösung von Methylhydrazin (0,43 g, 0,009 mol) in Toluol (15 ml) wurde über 20 min eine Lösung von 4-Butoxy-1-chlor-1,1-difluorbut-3-en-2-on (2 g, 0,009 mol) in Toluol (5 ml) getropft. Die Reaktion verläuft leicht exotherm. Das Reaktionsgemisch wird anschließend bei 0°C 30 min gerührt. Eine GC-Probe zeigt vollständigen Umsatz zu 2 neuen Produkten. Daneben wird n-Butanol detektiert. Anschließend wird das Reaktionsgemisch weitere 2 Tage bei Raumtemperatur gerührt. Die gelbe Reaktionslösung wird anschließend am Rotationsverdampfer eingeengt. Der ölige Rückstand (1,4g) wird in Toluol aufgenommen, mit einer Spatelspitze p-Toluolsulfonsäure (0,1g) versetzt und bei 80°C 1,5 h gerührt. Anschließend wird das Reaktionsgemisch nacheinander mit einer gesättigten wässrigen NaHCO₃-Lösung (20 mL) und mit Wasser (20 ml) gewaschen. Nach einengen der organischen Phase wurde als Rückstand ein gelbes Öl (0,3 g) erhalten.
EI-MS [m/z]: 166 [M]⁺; ¹H-NMR (500 MHz; CDCl₃): δ = 3,95 (s, 3H), 6,55 (s, 1H),
7,72 ppm (s, 1 H); ¹³C-NMR (125 MHz, CDCl₃): δ = 147,20, 104,35, 133,10, 39,65, 124,44 ppm.

### 10. Herstellung von 3-Difluorchlormethyl-N-methylpyrazol ausgehend von 4-Butoxy-1-chlor-1,1-difluorbut-3-en-2-on

Zu einer Lösung von 4-Butoxy-1-chlor-1,1-difluorbut-3-en-2-on (80%ig, 8 g, 0,03 mol) in Essigsäure (60 ml) wurde über 30 Minuten eine Lösung von Methylhydrazin (1,6 g, 0,034 mol) in Essigsäure (10 ml) getropft. Die Reaktion verläuft leicht exotherm. Die Lösung wurde ca. 16 h bei 25°C gerührt. Anschließend wurde die Reaktionslösung unter vermindertem Druck eingeengt (50°C / 30 mbar). Die GC-Analyse des Rückstands ergab ein Isomerenverhältnis von 11 : 1 zugunsten des 3-Isomeren (GC-Retentionszeiten: 4,3 min (5-Isomer); 8,2 min (3-Isomer)). Der Rückstand wurde mit Methyl-tert-butylether (MTBE, 20 ml) und Wasser (20 ml) versetzt. Nach Phasentrennung wurde die organische Phase über Natriumsulfat getrocknet, filtriert und unter vermindertem Druck von flüchtigen Bestandteilen befreit (50°C / 30 mbar). Der ölige Rückstand (3,7 g) wurde durch GC-Analyse untersucht. Die Flächen der beiden Isomere entsprachen 73 % der GC-Gesamtfläche und lagen in einem Verhältnis von 49 : 1 zugunsten des 3-Isomers vor. Die Ausbeute betrug etwa 58 %.
EI-MS (GC-MS) [m/z]: 166 [M]⁺.

### 11. Herstellung von 3-Difluorchlormethyl-NH-pyrazol ausgehend von 4-Butoxy-1-chlor-1,1-difluor-but-3-en-2-on

Zu einer Lösung von 4-Butoxy-1-chlor-1,1-difluorbut-3-en-2-on (80%ig, 8 g, 0,03 mol) in Essigsäure (60 ml) wurde eine Lösung von Hydrazin-Hydrat (1,7 g, 0,034 mol) in Essigsäure (10 ml) über 30 Minuten getropft. Die Reaktion verlief leicht exotherm. Das Reaktionsgemisch wurde etwa 16 h bei 25°C gerührt. Anschließend wurde das Reaktionsgemisch von flüchtigen Bestandteilen befreit (50°C / 30 mbar). Der Rückstand wurde mit Methyl-tert-butylether (MTBE, 20 ml) und Wasser (20 ml) versetzt. Nach Phasentrennung wurde die organische Phase über Natriumsulfat getrocknet, filtriert und vom Lösungsmittel befreit (50°C / 30 mbar). Der ölige Rückstand (5,1 g) bestand laut GC-Analyse zu 80%ige aus 3-Difluorchlormethyl-NH-pyrazol (GC-Retentionszeit:
11,9 min). Dies entspricht einer Ausbeute von 89 %.

### 12. Herstellung von 1-Methylpyrazol-3-carbaldehyd ausgehend von 4-(Dimethylamino)-1,1-dimethoxybut-3-en-2-on

Zu einer Lösung von NaOH (131,4g, 3,29 mol) in Wasser (1700 ml) wurde unter Kühlung Methylhydrazin (151,3 g, 3,29 mol) zugetropft. Anschließend wurde 4-(Dimethylamino)-1,1-dimethoxybut-3-en-2-on (569 g, 3,28 mol) bei Raumtemperatur zugetropft und über Nacht gerührt. Zur Reaktionsmischung wurde Salzsäure (36 %ig, 219,6 g, 2.17 mol) zugegeben und 4 h gerührt. Anschließend wurde das Reaktionsgemisch mit Natronlauge (10%ig) auf einen pH-Wert von 9,4 eingestellt. Nach viermaliger Extraktion mit Methylenchlorid (500 ml), wurden die organischen Phasen mit Wasser gewaschen, über Magnesiumsulfat getrocknet, filtriert und unter vermindertem Druck vom Lösungsmittel befreit. Der ölige Rückstand hatte laut GC-Analyse eine Reinheit von 73 %. Neben dem gewünschten 1-Methylpyrazol-3-carbaldehyd enthielt der Rückstand als Nebenprodukt 10,7 % des 5-Carbaldehyds. Dieses wurde durch Destillation unter vermindertem Druck (Übergangstemperatur: 55°C bei 2,5 bis 2,3 mbar) abgetrennt. Die Ausbeute an 1-Methylpyrazol-3-carbaldehyd betrug 53 %.
¹H-NMR (CDCl₃): δ = 4,05 (s, 3H) 6,8 (s, 1 H), 7,5 (s, 1 H), 9,95 ppm (s, 1 H).

### 13. Herstellung von 4-Brom-3-dichlormethyl-1-methylpyrazol ausgehend von 1-Methylpyrazol-3-carbaldehyd

### 13.1 Herstellung von 4-Brom-1-methylpyrazol-3-carbaldehyd durch Bromierung von 1-Methylpyrazol-3-carbaldehyd

Zu einer Lösung von 1-Methylpyrazol-3-carbaldehyd (40 g, 0,36 mol) in Dimethylformamid (DMF, 320 ml) wurde bei 3-5°C eine Lösung von N-Bromsuccinimid (NBS, 64,6 g, 0,36 mol) in DMF (160 ml) zugetropft (leicht exotherm). Das Reaktionsgemisch wurde anschließend auf Raumtemperatur erwärmt und weitere 2 h gerührt. Danach wurde das Reaktionsgemisch unter Rühren auf ein Gemisch aus Wasser (1400 ml) und Natronlauge (konz., 15ml) gegeben und viermal mit Methyl-tert-butylether (MTBE, 100 ml) extrahiert. Die organischen Phasen wurden vereint und mit ges. NaCl-Lsg. gewaschen, über Magnesiumsulfat getrocknet, filtriert und anschließend unter vermindertem Druck vom Lösungsmittel befreit. Man erhielt 4-Brom-1-methylpyrazol-3-carbaldehyd als Feststoff (42,9 g, 98 %ig laut GC-Analyse, Ausbeute 61,3 %).
¹H-NMR (CDCl₃): δ = 4,0 (s, 3H), 7,55 (s, 1 H), 9,95 ppm (s, 1H); ¹³C-NMR (CDCl₃):
δ = 40,3, 95,1, 133,0, 146,5, 184,6 ppm.

### 13.2 Herstellung von 4-Brom-3-dichlormethyl-1-methylpyrazol ausgehend von 4-Brom-1-methylpyrazol-3-carbaldehyd

4-Brom-1-methylpyrazol-3-carbaldehyd (4,0g, 0,02mol) wurden zusammen mit Triphenylphosphin (5,9 g, 0,022 mol) in Chlorbenzol (40 ml) vorgelegt. Anschließend wurde Thionylchlorid (2,6 g, 0,022 mol) bei Raumtemperatur zugetropft. Nach erfolgter Zugabe wurde das Reaktionsgemisch für eine Stunde auf 50°C erwärmt und über Nacht bei Raumtemperatur gerührt. Danach wurde Eis (40 g) zugegeben und zweimal mit Methylenchlorid (100 ml) extrahiert. Die organischen Phasen wurden über Magnesiumsulfat getrocknet, filtriert und unter vermindertem Druck vom Lösungsmittel befreit. Der ölige Rückstand wurde durch fraktionierende Destillation aufgetrennt. Das gewünschte Produkt (3,4 g, 95%ig, Ausbeute 66%) wurde bei einer Übergangstemperatur von 95°C bei 3,8 mbar isoliert.
¹H-NMR (CDCl₃): δ = 3,9 (s; 3H), 6,75 (s; 1 H), 7,4 ppm (s, 1H); ¹³C-NMR (CDCl₃):
δ = 39,9, 63,1, 95,1, 132,0, 147,8 ppm.

### 14. Herstellung von 4-Brom-3-dichlormethyl-1-methylpyrazol

### 14.1 Herstellung von (Z)-1,1-Dichlor-3,4-dibrombut-3-en-2-on aus 1,2-Dibromethylen und Dichloracetylchlorid

Zu einer Suspension aus 1,2-Dibromethylen (10,2 g, 0,055 mol) und AlCl₃ (4,88 g, 0,036 mol) wurde bei Raumtemperatur Dichloracetylchlorid (5,4 g, 0,036 mol) getropft (leicht exotherm). Das Reaktionsgemisch wurde kurz auf 55°C erhitzt und anschlie-βend aufgearbeitet. Das Reaktionsgemisch wurde auf Eiswasser (50 g) gegeben (schäumt und reagiert heftig) und dreimal mit Methylenchlorid (100 ml) extrahiert. Die organischen Phasen wurden bei 10°C mit gesättigter NaHCO₃-Lsg. gewaschen, über Magnesiumsulfat getrocknet, filtriert und unter vermindertem Druck vom Lösungsmittel befreit (30°C, 50 mbar). Man erhält 11,7g eines schwarzes Öls, das etwa zu 12 % (Z)-1,1-Dichlor-3,4-dibrombut-3-en-2-on und zu 29% (Z)-3-Brom-1,1,4-trichlorbut-3-en-2-on enthält. Die Ausbeute an (Z)-1,1-Dichlor-3,4-dibrombut-3-en-2-on entspricht 13%. ¹H-NMR (CDCl₃): δ = 6,6 (s, 1 H), 7,85 ppm (s, 1H); ¹³C-NMR (CDCl₃): δ = 66,7, 129,6, 135,9, 179,6 ppm.

### 14.2 Herstellung von 4-Brom-3-dichlormethyl-1-methylpyrazol aus (Z)-1,1-Dichlor-3,4-dibrombut-3-en-2-on

Eine Lösung von (Z)-1,1-Dichlor-3,4-dibrombut-3-en-2-on (50 %ig, 10 g, 0,0685 mol) in Diethylether (75 ml) wurde bei -40°C langsam zu einer etherischen Lösung von Methylhydrazin (4 g, 0,086mol) getropft. Anschließend wurde 2 h bei 0°C und bei Raumtemperatur über Nacht nachgerührt. Anschließend wurde die Reaktionsmischung filtriert und der Filterrückstand mit Methyl-tert-butylether (MTBE) gewaschen. Das Filtrat wurde unter vermindertem Druck vom Lösungsmittel befreit. Man erhielt 6,7 g eines braunen Öls mit einem Gehalt an 4-Brom-3-dichlormethyl-1-methylpyrazol von 53% (It. GC-Analyse). Dies entspricht einer Ausbeute von 86,4%.

### 15. Herstellung von 4-Brom-3-difluormethyl-1-methylpyrazol durch Fluorierung von 4-Brom-1-methylpyrazol-3-carbaldehyd

Zu einer Lösung von 4-Brom-1-methylpyrazol-3-carbaldehyd (22,1 g, 0,12mol) in Methylenchlorid (120 ml) wurde bei -20°C (Diethylamino)schwefeltrifluorid (DAST, 47,1 g, 0,29 mol) zugetropft. Das Reaktionsgemisch wurde bei Raumtemperatur über Nacht gerührt. Anschließend wurde das Reaktionsgemisch vorsichtig auf ein Eis-WasserGemisch (400 g) gegeben und zweimal mit Methylenchlorid (100 ml) extrahiert. Die organische Phase wurden zweimal mit Wasser (100 ml)und zweimal mit einer gesättigten wässrigen NaCl-Lsg. (100 ml) gewaschen, über Magnesiumsulfat getrocknet, filtriert und unter vermindertem Druck vom Lösungsmittel befreit. Der Rückstand wurde durch fraktionierende Destillation aufgetrennt. 4-Brom-3-diflluormethyl-1-methylpyrazol (Übergangstemperatur 51°C bei 2,5 mbar) wurde in einer Reinheit von 97 %
(GC-Analyse) mit einer Ausbeute von 24,2 g (48,7 %) isoliert.
¹H-NMR (CDCl₃): δ = 3,9 (s; 3H), 6,65 (t; 1 H), 7,45 ppm (s, 1 H).

### 16. Herstellung von 4-Brom-3-difluormethyl-1-methylpyrazol durch Halogenaustausch

4-Brom-3-dichlormethyl-1-methylpyrazol (3,0 g; 0,01 mol; 90 %ig laut GC-Analyse) wurde mit Triethylamin-Trishydrofluorid (25 g, 0,16 mol) bei 160°C unter Eigendruck (<1bar) 1 h gerührt. Nach Entspannen wird das Reaktionsgemisch auf Eis (200 g) gegeben, mit Natronlauge alkalisch gestellt und dreimal mit Methyl-tert-butylether (100 ml) extrahiert. Die vereinten organischen Phasen wurden nacheinander mit verd. Salzsäure (100 ml) und einer gesättigten wässrigen NaCl-Lsg. (100 ml) gewaschen, über Magnesiumsulfat getrocknet und unter vermindertem Druck vom Lösungsmittel befreit. 4-Brom-3-difiluormethyl-1-methylpyrazol wurde in einer Reinheit von 81,3 %
(GC-Analyse) mit einer Ausbeute von 2,0 g (70 %) isoliert.
¹H-NMR (DMSO-d₆): δ = 3,85 (s, 3H), 6,95 (t, 1 H), 8,1 ppm (s, 1 H).

### 17. Herstellung von 3-Diflluormethyl-1-methylpyrazol aus 1-Methylpyrazol-3-carbaldehyd

Zu einer Lösung von 1-Methylpyrazol-3-carbaldehyd (20 g, 0,18mol) in Methylenchlorid (200 ml) wurde bei -20°C (Diethylamino)schwefeltrifluoride (DAST, 87,8 g, 0,54 mol) zugetropft. Das Reaktionsgemisch wurde bei Raumtemperatur über Nacht gerührt. Anschließend wurde das Reaktionsgemisch vorsichtig auf Eis-Wasser (400 g) gegeben und zweimal mit Methylenchlorid (je 100 ml) extrahiert. Die vereinte organische Phase wurde zweimal mit Wasser (je 100 ml) und zweimal mit NaCl-Lsg. (je 100 ml) gewaschen, über Magnesiumsulfat getrocknet und unter vermindertem Druck vom Lösungsmittel befreit. Der Rückstand wurde durch fraktionierende Destillation aufgetrennt. 3-Diflluormethyl-1-methylpyrazol (16,5 g, Übergangstemp. 141°C bei 570 mbar) wurde in einer Reinheit von 95 % isoliert. Dies entspricht einer Ausbeute von 66,8%.

### 18. Herstellung von 3-Difluormethyl-NH-pyrazol aus Propargylaldehyddimethylacetal

### 18.1 Herstellung von 1,1-Difluor-4,4-dimethoxy-but-2-in

Zu einer Lösung von Propargylaldehyddimethylacetal (52,8 g, 0,5 mol) in trockenem Tetrahydrofuran (THF, 1000 ml) wurde bei -70°C eine n-Buthyllithium-Lösung (2,5 M, in Hexan, 260 ml, 0,65 mol) zugetropft. Anschließend wurde das Reaktionsgemisch 30 min. bei -70°C gerührt und auf -100°C abgekühlt. Bei dieser Temperatur wurde Chlordifluormethan (216,6 g, 2,5 mol) in das Reaktionsgemisch eingeleitet (stark exotherm). Das Reaktionsgemisch wurde langsam auf -50°C erwärmt und 1 h bei dieser Temperatur gerührt. Danach wurde das Reaktionsgemisch mit wässriger ges. NH₄Cl-Lsg. (400 ml) versetzt, auf 0°C erwärmt, mit einem Wasser/MTBE-Gemisch (500 ml) versetzt und die wässrige Phase abgetrennt. Die wässrige Phase wurde zweimal mit MTBE (200 ml) extrahiert. Die vereinten organischen Phasen wurden zweimal mit verd. Salzsäure (100 ml) gewaschen, über Magnesiumsulfat getrocknet, filtriert und unter vermindertem Druck vom Lösungsmittel befreit. Der Rückstand wurde durch fraktionierende Destillation aufgetrennt. 1,1-Difluor-4,4-dimethoxy-but-2-in (46,7 g; Übergangstemp. 50-52°C bei 30mbar) wurde in einer Reinheit von 96 % (It. GC-Analyse) erhalten. Dies entspricht einer Ausbeute von 65 %. Weiteres Produkt wurde in der Kühlfalle und im abdestillierten Lösungsmittel gefunden. Die nichtisolierte Ausbeute beträgt somit 71 %.
¹H-NMR (CDCl₃): δ = 3,4 (s, 6H), 5,2 (s, 1H), 6,25 ppm (t, 1H);
¹³C-NMR (CDCl₃): δ = 14,9, 61,4, 75,9, 83,9, 90,7, 103,4 ppm.

### 18.2 Herstellung von 3-Difluormethyl-NH-pyrazol aus 1,1-Difluor-4,4-dimethoxy-but-2-in

Eine Suspension von 1,1-Difluor-4,4-dimethoxy-but-2-in (98%ig, 0,3g, 0,002mol) und Hydrazinsulfat (0,2 g, 0,0012 mol) in 2,5ml Eisessig wurde bei 55°C 2 h gerührt. Das Reaktionsgemisch wurde auf Raumtemperatur erwärmt, mit einem Gemisch aus Essigester und Wasser (10 ml) versetzt und mit verd. Natronlauge neutralisiert. Nach Phasentrennung wurde die organische Phase zweimal mit Wasser (100 ml) und einmal mit verd. wässriger NaCl-Lsg. gewaschen, über Magnesiumsulfat getrocknet, filtriert und unter vermindertem Druck vom Lösungsmittel befreit. Der rote ölige Rückstand enthielt zu 70 % 3-Difluormethyl-NH-pyrazol (It. GC- und ¹H-NMR-Analyse). Die Ausbeute entspricht 75%.
¹H-NMR (CDCl₃): δ = 6,65 (s, 1 H), 6,8 (t, 1 H), 7,68 (s, 1 H), 10,2 ppm (s, 1 H).

### 19. Herstellung von 3-Difluormethyl-1-methylpyrazol aus 1,1-Difluor-4,4-dimethoxy-but-2-in

Eine Suspension von 1,1-Difluor-4,4-dimethoxy-but-2-in (98 %ig, 1 g, 6,5 mmol), wässrigem Methylhydrazin (25 %ig, 1,7g, 13,0 mmol) und konz. Schwefelsäure (0,7 g, 6,5 mmol) in Eisessig (10 ml) wurde 6 h bei 100°C gerührt. Das Reaktionsgemisch wurde auf Raumtemperatur erwärmt, mit einem Essigester/Wasser-Gemisch (10 ml) versetzt und mit verd. Natronlauge neutralisiert. Nach Phasentrennung wurde die wässrige Phase dreimal mit Essigester extrahiert. Die gesammelten organischen Phasen wurden zweimal mit Wasser (100 ml) und einmal mit verd. wässriger NaCl-Lsg. gewaschen, über Magnesiumsulfat getrocknet und unter vermindertem Druck vom Lösungsmittel befreit. Der rote ölige Rückstand enthielt zu 61,8 % 3- und 5-Diflluormethyl-1-methylpyrazol (It. GC- und ¹H-NMR-Analyse). Die gemeinsame Ausbeute beider Isomere betrug 60,5 %. Die Ausbeute an 3-Difluormethyl-1-methylpyrazol betrug 29,2%.

### 20. Herstellung von 1,1-Difluor-4,4-diethoxy-but-2-in

Analog zur Herstellung von 1,1-Difluor-4,4-dimethoxy-but-2-in in Beispiel 17.1 wurde ausgehend vom Diethylacetal 1,1-Difluor-4,4-diethoxy-but-2-in hergestellt. Die Ausbeute betrug 66,2 %.
¹H-NMR (CDCl₃): δ = 1,25 (t, 6H), 3,7 (m, 4H), 5,3 (s, 1 H), 6,25 ppm (t, 1 H).
¹³C-NMR (CDCl₃): δ = 52,9, 76,5, 83,2, 92,6, 103,5 ppm.

### 21. Herstellung von 4-Chlor-3-difluormethyl-1-methylpyrazol

### 21.1 Herstellung von (Z)-1,1,3,4-Tetrachlorbut-3-en-2-on

Zu einer Suspension von AlCl₃ (45 g, 0,339 mol) in 1,2-Dichlorethylen (49,3 g, 0,508 mol) wurde bei Raumtemperatur Dichloracetylchlorid (50 g, 0,339 mol) zugetropft (leicht exotherm). Das Reaktionsgemisch wurde 3 h unter Rückflussbedingungen und anschließend über Nacht bei Raumtemperatur gerührt. Anschließend wurde das Reaktionsgemisch in Eiswasser (500 g) eingerührt (schäumt und reagiert heftig) und dreimal mit Methylenchlorid (200 ml) extrahiert. Die vereinigten organischen Phasen wurden einmal bei 10°C mit einer ges. wässrigen NaHCO₃-Lsg. gewaschen, über Magnesiumsulfat getrocknet und bei einer Badtemperatur von 70 °C bei Normaldruck vom Lösungsmittel befreit. Der Rückstand wurde bei 50 mbar und 70 °C andestilliert. Man erhält 46,5g eines schwarzes Öls, das einen Gehalt an (Z)-1,1,3,4-Tetrachlorbut-3-en-2-on von etwa 70 % hat und 25% einer Nebenkomponente enthält, bei der es sich um (Z)-1,1,1,4-Tetrachlorbut-3-en-2-on handelt. Die Ausbeute an (Z)-1,1,3,4-Tetrachlorbut-3-en-2-on beträgt somit 38 %.

### (Z)-1,1,3,4-Tetrachlorbut-3-en-2-on:

¹H-NMR (CDCl₃): δ = 6,6 (s, 1 H), 7,85 ppm (s, 1 H).
¹³C-NMR (CDCl₃): δ = 66,7, 129,6, 135,9, 179,6 ppm.

### (Z)-1,1,1,4-Tetrachlorbut-3-en-2-on:

¹H-NMR (CDCl₃): δ = 7,15 (s; 1 H), 7,7 ppm (s, 1 H).
¹³C-NMR (CDCl₃): δ = 95,2,123,2,143,2,177,5 ppm.

### 21.2 Herstellung von 4-Chlor-3-dichlormethyl-1-methylpyrazol

Eine Lösung von (Z)-1,1,3,4-Tetrachlorbut-3-en-2-on (60%ig, 10 g, 28,8 mmol) in Diethylether (75 ml) wurde bei -40 °C zu einer etherischen Lösung von Methylhydrazin (4 g, 86 mmol) langsam zugetropft. Anschließend wurde das Reaktionsgemisch 2 h bei 0 °C und über Nacht bei Raumtemperatur gerührt. Das Reaktionsgemisch wurde filtriert und der Filterkuchen mit Methyl-tert-Butylether (MTBE) nachgewaschen. Das Filtrat wurde unter vermindertem Druck von flüchtigen Bestandteilen befreit. Das als Rückstand erhaltene braune Öl (10 g) hatte gemäß GC-Analytik einen Gehalt an 4-Chlor-3-dichlormethyl-1-methylpyrazol von 70%. Der Rückstand wurde einer fraktionierenden Destillation unter vermindertem Druck (3,5 mbar) unterworfen. Durch Destillation bei 90 - 100°C (Bad: 157-190°C) wurden zwei Fraktionen, die 4-Chlor-3-dichlormethyl-1-methylpyrazol enthalten, erhalten (Ausbeute 70% d. Th.). Diese enthielten als weitere Komponente 1-Methyl-3-trichlormethylpyrazol.

### 4-Chlor-3-dichlormethyl-1-methylpyrazol:

¹H-NMR (CDCl₃): δ = 3,9 (s, 3H), 6,75 (s, 1 H), 7,35 ppm(s, 1 H).
¹³C-NMR (CDCl₃): δ = 39,9, 146,3, 107,9, 129,7, 62,4 ppm.

### 1-Methyl-3-trichlormethylpyrazol:

¹H-NMR (CDCl₃): δ = 3,95 (s, 3H), 6,6 (s, 1 H), 7,35 ppm (s, 1 H).
¹³C-NMR (CDCl₃): δ = 39,5, 154,8, 104,4, 131,5, 90,7 ppm (CCl₃).

### 21.3 Herstellung von 4-Chlor-3-difluormethyl-1-methylpyrazol

4-Chlor-3-dichlormethyl-1-methylpyrazol (2,2 g, 0,01 mol) wurde mit Triethylamin-trishydrofluorid (25 g, 0,16 mol) unter Eigendruck (<1 bar) 1 h bei 160 °C gerührt. Nach Entspannen wird die Reaktionsmischung auf Eis (100 g) gegeben, mit einer wässrigen NaOH-Lösung alkalisch gestellt und dreimal mit Methy-tert-butylether (100 ml) extrahiert. Die vereinigten organischen Phasen wurden nacheinander mit verd. Salzsäure (100 ml) und einer wässrigen NaCl-Lsg. (100 ml) gewaschen, über Magnesiumsulfat getrocknet, filtriert und unter vermindertem Druck vom Lösungsmittel befreit. Als Rückstand wurde 4-Chlor-3-difluormethyl-1-methylpyrazol (1,5 g) erhalten, das gemäß GC-Analytik eine Reinheit von 99 % aufwies. Dies entspricht einer Ausbeute an 4-Chlor-3-difluormethyl-1-methylpyrazol von 89 %.

¹H-NMR (DMSO-d₆): δ = 3,85 (s, 3H), 7,0 (t, 1 H), 8,1 ppm (s, 1 H).

### 22. Herstellung von 3-Difluormethyl-NH-pyrazol aus 3-Dichlormethyl-NH-pyrazol

Analog zur Herstellung von 4-Brom-3-difluormethyl-1-methylpyrazol durch Halogenaustausch (Beispiel 15), wurde 3-Dichlormethyl-NH-pyrazol (1,4 g, 0,01 mol; It. GC 75%ig) mit Triethylamin-Trishydrofluorid (50 g, 0,31 mol) bei 80 °C unter Eigendruck (<1bar) 13 h gerührt. Nach Entspannen wurde das Reaktionsgemisch auf Eis (200 g) gegeben, mit Natronlauge alkalisch gestellt und dreimal mit Methyl-tert-Butylether (MTBE, 100 ml) extrahiert. Die vereinigten organischen Phasen wurden nacheinander mit verd. Salzsäure (100 ml) und einer gesättigten wässrigen NaCl-Lsg. (100 ml) gewaschen, über Magnesiumsulfat getrocknet, filtriert und unter vermindertem Druck vom Lösungsmittel befreit. 4-Brom-3-difluormethyl-1-methylpyrazol wurde in einer Reinheit von 87 % (GC-Analyse) mit einer Ausbeute von 0,7 g (44 %) isoliert.
¹H-NMR (DMSO-d₆): δ = 3,85 (s, 3H), 6,95 (t, 1 H), 8,1 ppm (s, 1 H).

### 23. Herstellung von N-(2-(2-cyclopropylcyclopropyl)phenyl)-3-difluormethyl-1-methylpyrazol-4-carbonsäureamid in Gegenwart von 2,2-Dimethyl-1,3-bis-(diphenylphosphino)propan (Pepstar)

Eine Lösung von Pd(PhCN)₂Cl₂ (0.014 mmol) und Pepstar (0.042 mmol) in Dimethylformamid (DMF, 3 ml) wurde 30 Minuten bei Raumtemperatur gerührt. Die Lösung enthaltend den präformierten Katalysator wurde in einen inertisierten Autoklaven überführt. Diazabicycloundecen (DBU, 3.08 mmol), sowie Lösungen von 3-Difluormethyl-1-methyl-4-brompyrazol (2.8 mmol) und 2-(2-Cyclopropylcyclopropyl)anilin (2.8 mmol) in DMF (je 3.25 ml) wurden im CO-Gegenstrom zu der Lösung des Katalysators in den Autoklaven überführt. Das Reaktionsgemisch wurde 10 min bei Raumtemperatur und weitere 16 h bei 150 °C und bei einem CO-Druck von 15 bar geschüttelt. Der Umsatz bezogen auf Brompyrazol betrug 97.6 % bei einer Selektivität von 83.4 %. Die Zusammensetzung des Reaktionsaustrags war laut GC-Analyse wie folgt: DMF 33.51 FI.-%, Brompyrazol 0.83 FI.-%, DBU 31.65 FI.-%, 2-(2-Cyclopropylcyclopropyl)anilin 0.0 FI.-%, N-(2-(2-cyclopropylcyclopropyl)phenyl)-3-difluormethyl-1-methylpyrazol-4-carbonsäureamid 28.37 FI.-%.

Die Aufarbeitung erfolgte wie in Beispiel 2.i) beschrieben und ergab eine isolierte Ausbeute an N-(2-Cyclopropylcyclopropyl)-phenyl)-3-difluormethyl-1-methylpyrazol-4-carbonsäureamid von 81.4 %.

### 24. Herstellung von N-(2-(3,4-Dichlorphenyl)-4-fluorphenyl)-3-difluormethyl-1-methylpyrazol-4-carbonsäureamid in Gegenwart von 2,2-Dimethyl-1,3-bis-(diphenylphosphino)propan (Pepstar)

Eine Lösung von Pd(PhCN)₂Cl₂ (0.014 mmol) und Pepstar (0.042 mmol) in Dimethylformamid (DMF, 3 ml) wurde 30 Minuten bei Raumtemperatur gerührt. Die Lösung enthaltend den präformierten Katalysator wurde in einen inertisierten Autoklaven überführt. Diazabicycloundecen (DBU, 3.08 mmol), sowie Lösungen von 3-Difluormethyl-1-methyl-4-brompyrazol (2.8 mmol) und 2-(3,4-Dichlorphenyl)-4-fluoranilin (2.8 mmol) in DMF (je 3.25 ml) wurden im CO-Gegenstrom zu der Lösung des Katalysators in den Autoklaven überführt. Das Reaktionsgemisch wurde 10 min bei Raumtemperatur und weitere 16 h bei 150 °C und bei einem CO-Druck von 15 bar geschüttelt.
Der Umsatz bezogen auf 4-Brompyrazol betrug 97.2 % bei einer Selektivität von 56.8 %. Die Zusammensetzung des Reaktionsaustrags war laut GC-Analyse wie folgt: DMF 32.65 FI.-%, Brompyrazol 1.50 FI.-%, DBU 14.64 FI.-%, 2-(3,4-Dichlorphenyl)-4-fluoranilin 3.29 FI.-%, N-(2-(3,4-Dichlorphenyl)-4-fluorphenyl)-3-difluormethyl-1-methylpyrazol-4-carbonsäureamid 29.11 FI.-%.

Die Aufarbeitung erfolgte wie in Beispiel 2.i) beschrieben und ergab eine isolierte Ausbeute an N-(2-(3,4-Dichlorphenyl)-4-fluorphenyl)-3-difluormethyl-1-methylpyrazol- 4-carbonsäureamid von 55.2 %.

### 25. Herstellung von N-(9-Isopropylbenzonorbornen-5-yl)-3-difluormethyl-1-methylpyrazol-4-carbonsäureamid in Gegenwart von 2,2-Dimethyl-1,3-bis-(diphenylphosphino)propan (Pepstar)

Eine Lösung von Pd(PhCN)₂Cl₂ (0.0375 mmol) und Pepstar (0.1125 mmol) in Dimethylformamid (DMF, 10 ml) wurde 30 Minuten bei Raumtemperatur gerührt. Die Lösung enthaltend den präformierten Katalysator wurde in einen inertisierten Autoklaven überführt. Diazabicycloundecen (DBU, 16.5 mmol), sowie Lösungen von 3-Difluormethyl-1-methyl-4-brompyrazol (16.5 mmol) und 5-Amino-9-isopropylbenzonorbornen (15 mmol) in DMF (je 25 ml) wurden im CO-Gegenstrom zu der Lösung des Katalysators in den Autoklaven überführt. Das Reaktionsgemisch wurde 24 h bei 150 °C und bei einem CO-Druck von 15 bar gerührt.

Der Umsatz bezogen auf das Brompyrazol war annähernd quantitativ bei einer Selektivität von 38 %. Die Zusammensetzung des Reaktionsaustrags laut GC-Analyse war wie folgt: DMF 73.8 FI.-%, Brompyrazol 0.02 FI.-%, DBU 8.2 FI.-%, Aminoisopropylbenzonorbornen 7.1 FI.-% und N-(9-Isopropylbenzonorbornen-5-yl)-3-difluormethyl-1-methylpyrazol-4-carbonsäureamid 4.08 FI.-%.

Die Aufarbeitung erfolgte wie in Beispiel 2.i) beschrieben und ergab eine isolierte Ausbeute an N-(9-Isopropylbenzonorbornen-5-yl)-3-difluormethyl-1-methylpyrazol-4-carbonsäureamid von 38 %.

### 26. Herstellung von 3-Difluormethyl-N-methylpyrazol durch Halogenaustausch

3-Dichlormethyl-N-methylpyrazol (40,0 g; 0,21 mol; 84,8 %ig laut GC-Analyse), enthaltend als Nebenkomponente 5-Dichlormethyl-N-methylpyrazol, wurde mit Triethylamin-Trishydrofluorid (199 g, 1,23 mol) bei 160 °C unter Eigendruck (<1bar) 1 h gerührt. Nach Entspannen wurde das Reaktionsgemisch auf Eis (500 g) gegeben, mit Natronlauge alkalisch gestellt und dreimal mit Methyl-tert-butylether (100 ml) extrahiert. Die organischen Phasen wurden vereint, nacheinander mit Salzsäure (verdünnt, 100 ml) und einer gesättigten wässrigen NaCl-Lösung (100 ml) gewaschen, über Magnesiumsulfat getrocknet und unter vermindertem Druck vom Lösungsmittel befreit. Als Rückstand wurde 3-Difluormethyl-1-methylpyrazol (24,8 g, Ausbeute: 78 %) in einer Reinheit von 85,4 % (laut GC-Analyse), enthaltend als Nebenkomponente 5-Difluormethyl-N-methylpyrazol, erhalten.

### 27. Selektive Bromierung von 3-Difluormethyl-N-methylpyrazol gegenüber 5-Difluormethyl-N-methylpyrazol

Zu einer Lösung eines Isomerengemisches aus 3-Difluormethyl-N-methylpyrazol und 5-Difluormethyl-N-methylpyrazols (2,8 g; Verhältnis laut GC-Analyse: 10 : 1) in Methylenchlorid (20 ml) wird Brom (3,2 g, ca. 1 Äq.) bei einer Temperatur von 25 °C zugetropft. Das Reaktionsgemisch wird insgesamt 5 h bei 25°C gerührt. Laut GC-Kontrolle wurde 3-Difluormethyl-N-methylpyrazol zu 67 % umgesetzt, während 5-Difluormethyl-N-methylpyrazols weitgehend unumgesetzt im Reaktionsgemisch vorliegt. Nach einer weiteren Zugabe von Brom (1,6 g, ca. 0.5 eq) wurde das Reaktionsgemischweitere 22 h bei 25°C gerührt. Laut GC-Kontrolle wurde 3-Difluormethyl-N-methylpyrazol zu 96 % umgesetzt, während 5-Difluormethyl-N-methylpyrazol weitgehend unumgesetzt im Reaktionsgemisch vorliegt. Das Reaktionsgemisch wurde mit Methylenchlorid (20 ml) verdünnt und mit einer wässrigen Lösung von Natriumthiosulfat (0,1 M, 70 ml) gewaschen. Die so erhaltene organischen Phase enthielt neben 4-Brom-3-difluormethyl-N-methylpyrazol das nicht umgesetzte 5-Difluormethyl-N-methylpyrazols. 4-Brom-3-difluormethyl-N-methylpyrazol wurde durch fraktionierte Destillation in großer Reinheit isoliert.

### 28. Selektive Bromierung von 3-Difluormethyl-N-methylpyrazol mit NBS

Zu einer Lösung von 3-Difluormethyl-N-methylpyrazol (49,3 g, 0,3 mol; 81,4 %ig It. GC-Analyse), enthaltend als Nebenkomponente 5,2 % an 5-Difluormethyl-N-methylpyrazol, bezogen auf die Gesamtmenge des Isomerengemischs, in Dimethylformamid (DMF, 100 ml) wurde bei 3 bis 5 °C eine Lösung von N-Bromsuccinimid (NBS, 54 g, 0,3 mol) in DMF (100 ml) zugetropft. Das Reaktionsgemisch wurde anschließend auf Raumtemperatur erwärmt und weitere 12 h gerührt. Danach wurde das Reaktionsgemisch unter Rühren auf Wasser (1000 ml) gegossen und zweimal mit Methyl-tert-butylether (MTBE, 300 ml) extrahiert. Die organischen Phasen wurden vereint, mit einer gesättigten wässrigen Lösung von NaCl gewaschen, über Magnesiumsulfat getrocknet, filtriert und anschließend unter vermindertem Druck vom Lösungsmittel befreit. 4-Brom-3-difluormethyl-N-methylpyrazol wurde als Öl (76,6 g; Reinheit: 74,2 % laut GC-Analyse), enthaltend 4,5 % an unumgesetztem 5-Difluormethyl-N-methylpyrazol, erhalten. Dies entspricht einer Ausbeute von 89 %.

### 29. Selektive Chlorierung von 3-Difluormethyl-N-methylpyrazol mit NCS

Zu einer Lösung von 3-Difluormethyl-N-methylpyrazol (14,9g, 0,09 mol, 83,7%ig It. GC-Analyse), enthaltend als Nebenkomponente 4,1 % an 5-Difluormethyl-N-methylpyrazol bezogen auf die Gesamtmenge des Isomerengemischs, in Dimethylformamid (DMF, 30 ml) wurde bei 0 - 5°C eine Lösung von N-Chlorsuccinimid (NCS, 12,6 g, 0,09 mol) in DMF (70 ml) zugetropft. Das Reaktionsgemisch wurde anschließend auf 60 °C erwärmt und weitere 12 h gerührt. Danach wurde das Reaktionsgemisch unter Rühren auf Wasser (1000 ml) gegossen und zweimal mit Methyl-tert-butylether (MTBE, 150 ml) extrahiert. Die organischen Phasen wurden vereint, mit einer gesättigten, wässrigen Lösung von NaCl gewaschen, über Magnesiumsulfat getrocknet, filtriert und anschlie-βend unter vermindertem Druck vom Lösungsmittel befreit. 4-Chlor-3-difluormethyl-N-methylpyrazol wurde als Öl (17,6 g; Reinheit: 78,4 % laut GC-Analyse); enthaltend 3,7 % an unumgesetzten 5-Difluormethyl-N-methylpyrazols, erhalten. Dies entspricht einer Ausbeute von 88 %.

## Patentansprüche

1. Verfahren zur Herstellung von Verbindungen der Formel (I), 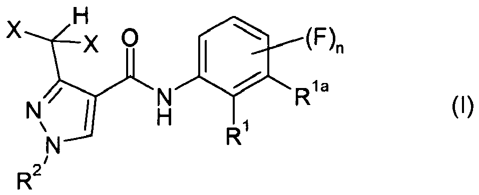 worin
R¹ für Phenyl oder C₃-C₇-Cycloalkyl steht, die unsubstituiert sind oder 1, 2 oder 3 Substituenten R^{a1} aufweisen, die unabhängig voneinander ausgewählt sind unter Halogen, C₁-C₈-Alkyl, C₁-C₄-Alkoxy, C₁-C₄-Alkylthio, C₁-C₄-Haloalkyl, C₁-C₄-Haloalkoxy, C₁-C₄-Haloalkylthio und C₃-C₇-Cycloalkyl,
R^{1a} für Wasserstoff oder Fluor steht, oder R^{1a} zusammen mit R¹ für C₃-C₅-Alkandiyl oder C₅-C₇-Cycloalkandiyl steht, wobei C₃-C₅-Alkandiyl und C₅-C₇-Cycloalkandiyl unsubstituiert sind oder 1, 2 oder 3 Substituenten ausgewählt unter C₁-C₄-Alkyl aufweisen,
R² für Wasserstoff, C₁-C₆-Alkyl, C₂-C₆-Alkenyl, C₂-C₆-Alkinyl oder C₁-C₄-Alkoxy-C₁-C₂-alkyl steht,
X für F oder Cl steht und
n für 0, 1, 2 oder 3 steht;
umfassend
A) die Bereitstellung einer Verbindung der Formel (II) 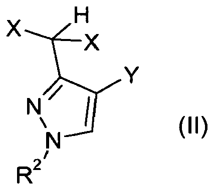 worin
X für F oder Cl steht,
Y für Cl oder Br steht und
R² eine der zuvor gegebenen Bedeutungen besitzt; und
B) die Umsetzung einer Verbindung der Formel (II) mit Kohlenmonoxid und einer Verbindung der Formel (III), 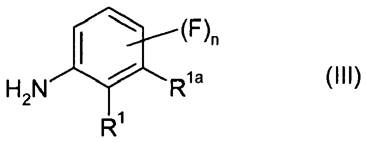 worin R¹, R^{1a} und n eine der zuvor gegebenen Bedeutungen besitzen,
in Gegenwart eines Palladium-Katalysators.

2. Verfahren nach Anspruch 1, worin die Verbindung der Formel (I) ausgewählt ist unter Verbindungen der Formel (I.1), 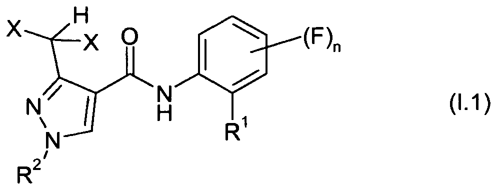 worin R¹, R², X und n unabhängig voneinander eine der für die Verbindungen der Formel (I) gegebenen Bedeutungen aufweisen,
und worin die Verbindung der Formel (III) ausgewählt ist unter Verbindungen der Formel (III.1), 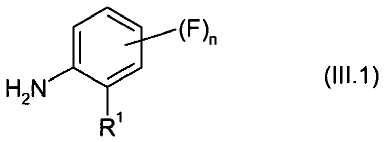 worin R¹ und n unabhängig voneinander eine der für die Verbindungen der Formel (III) gegebenen Bedeutungen aufweisen.

3. Verfahren nach einem der Ansprüche 1 oder 2, worin die Bereitstellung einer Verbindung der Formel (II), worin Y für Br oder Cl steht,
A.1) die Bereitstellung einer Verbindung der Formel (IV), 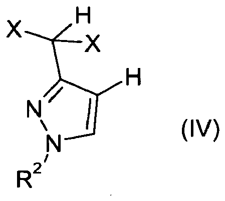 worin
X für Chlor oder Fluor steht und
R² eine der zuvor gegebenen Bedeutungen besitzt,
A.2) gegebenenfalls für den Fall, dass X in der Verbindung der Formel (IV) für Chlor steht, einen Halogenaustausch gegen Fluor und
A.3) die Chlorierung oder Bromierung einer Verbindung der Formel (IV) umfasst.

4. Verfahren nach Anspruch 3, worin die Bereitstellung einer Verbindung der Formel (IV)
A.1.1a) die Bereitstellung einer Verbindung der Formel (V), 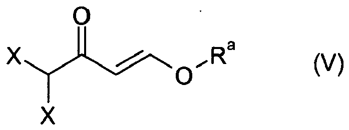 worin
X für Cl oder Fluor steht und
R^{a} für C₁-C₆-Alkyl oder C₂-C₆-Alkenyl steht, und
A.1.2a) die Umsetzung einer Verbindung der Formel (V) mit einer Hydrazinverbindung der Formel R²HN-NH₂, worin R² eine der zuvor gegebenen Bedeutungen besitzt, umfasst.

5. Verfahren nach Anspruch 3, worin die Bereitstellung einer Verbindung der Formel (IV)
A.1.1b) die Bereitstellung einer Verbindung der Formel (VI), 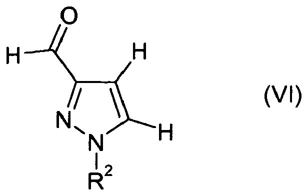 worin R² eine der zuvor gegebenen Bedeutungen besitzt; sowie
A.1.2b) die Umsetzung einer Verbindung der Formel (VI) durch Überführung der Carbonylgruppe in eine Dihalomethylgruppe mit einem geeigneten Halogenierungsmittel, umfasst.

6. Verfahren nach Anspruch 3, worin die Bereitstellung einer Verbindung der Formel (IV)
A.1.1c) die Deprotonierung eines Propargylaldehyd-Acetals,
A.1.2c) eine Umsetzung des deprotonierten Propargylaldehyd-Acetals aus Schritt A.1.1 c mit einer Verbindung der Formel CHX₂Cl, worin X für F oder Cl steht, und
A.1.3c) das anschließende Überführen des Umsetzungsprodukts aus Schritt A.1.2c in eine Verbindung der Formel (IV) mit einer Hydrazinverbindung der Formel R²HN-NH₂, worin R² eine der zuvor gegebenen Bedeutungen besitzt, umfasst.

7. Verfahren nach Anspruch 3, worin die Bereitstellung einer Verbindung der Formel (IV), worin X für Fluor steht,
A.1.1d) die Bereitstellung einer Verbindung der Formel (VII), 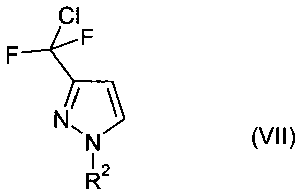 worin R² eine der zuvor gegebenen Bedeutungen besitzt; und
A.1.2d) die Dechlorierung der Verbindung der Formel (VII) umfasst.

8. Verfahren nach Anspruch 3, worin die Bereitstellung einer Verbindung der Formel (IV)
A.1.1e) die Bereitstellung einer Verbindung der Formel (VIII), 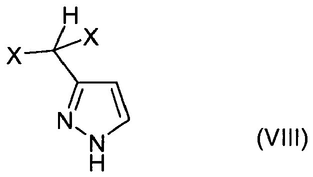 worin X für Cl oder Fluor steht und
A.1.2e) die N-Alkylierung der Verbindung der Formel (VIII) umfasst.

9. Verfahren nach Anspruch 8, worin die Bereitstellung einer Verbindung der Formel (VIII), worin X für F steht,
die Bereitstellung einer Verbindung der Formel (IX) 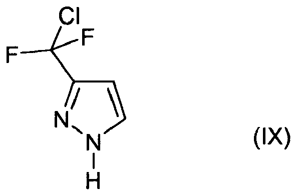 sowie die Dechlorierung der Verbindung der Formel (IX) umfasst.

10. Verfahren nach einem der Ansprüche 1 oder 2, worin die Bereitstellung einer Verbindung der Formel (II)
A.1') die Bereitstellung einer Verbindung der Formel (VI), wie in Anspruch 5 definiert,
A.2') die Chlorierung oder Bromierung einer Verbindung der Formel (VI) in 4-Position des Pyrazols, wobei man eine Verbindung der Formel (X) erhält, 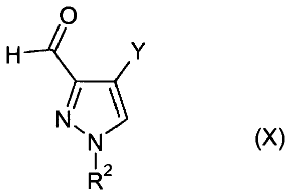 worin Y für Cl oder Br steht, sowie
A.3') die Überführung der Carbonylgruppe der Verbindung der Formel (X) in eine Dihalomethylgruppe umfasst.

11. Verfahren nach einem der Ansprüche 1 oder 2, worin die Bereitstellung einer Verbindung der Formel (II), worin Y für Cl steht,
A.1") die Bereitstellung einer Verbindung der Formel (XI), 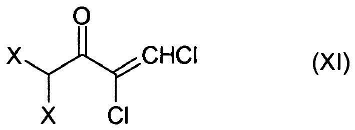 worin X für F oder Chlor steht,
A.2") die Umsetzung einer Verbindung der Formel (XI) mit einem geeigneten Hydrazinderivat der Formel R²HN-NH₂ zu einer Verbindung der allgemeinen Formel (II), worin X für F oder Cl steht, Y für Cl steht und R² eine der zuvor gegebenen Bedeutungen besitzt, sowie
A.3") gegebenenfalls für den Fall, dass X in der Verbindung der Formel (II) für Chlor steht, einen Halogenaustausch gegen Fluor umfasst.

12. Verfahren nach einem der vorhergehenden Ansprüche, worin X in den Verbindungen der allgemeinen Formel (I) für Fluor steht.

13. Verbindungen der allgemeinen Formel (II), wie in Anspruch 1 definiert.

14. Verbindungen der allgemeinen Formel (IV), wie in Anspruch 3 definiert.

15. Verbindungen der allgemeinen Formel (VII), wie in Anspruch 7 definiert.

16. Verbindungen der allgemeinen Formel (VIII), wie in Anspruch 8 definiert.

17. Verbindungen der allgemeinen Formel (IX), wie in Anspruch 9 definiert.

18. Verfahren zur Herstellung von Verbindungen der Formel (II), wie in Anspruch 13 definiert, umfassend,
A.1) die Bereitstellung einer Verbindung der Formel (IV) 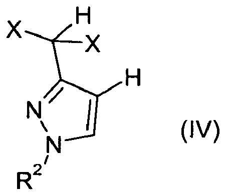 worin
X für Chlor oder Fluor und
R² eine der zuvor gegebenen Bedeutungen besitzt, steht,
A.2) gegebenenfalls für den Fall, dass X in der Verbindung der Formel (IV) für Chlor steht, einen Halogenaustausch gegen Fluor und
A.3) die Chlorierung oder Bromierung einer Verbindung der Formel (IV) umfasst.

## Claims

1. A process for preparing compounds of the formula (I) 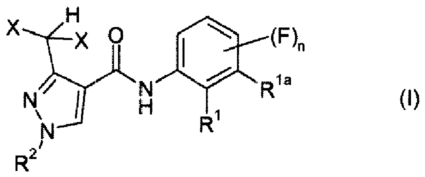 in which
R¹ is phenyl or C₃-C₇-cycloalkyl which are unsubstituted or have 1, 2 or 3 substituents R^{a1} independently of one another selected from the group consisting of halogen, C₁-C₈-alkyl, C₁-C₄-alkoxy, C₁-C₄-alkylthio, C₁-C₄-haloalkyl, C₁-C₄-haloalkoxy, C₁-C₄-haloalkylthio and C₃-C₇-cycloalkyl,
R^{1a} is hydrogen or fluorine, or
R^{1a} together with R¹ is C₃-C₅-alkandiyl or C₅-C₇-cycloalkanediyl, where C₃-C₅-alkanediyl and C₅-C₇-cycloalkanediyl are unsubstituted or have 1, 2 or 3 substituents selected from the group consisting of C₁-C₄-alkyl radicals,
R² is hydrogen, C₁-C₆-alkyl, C₂-C₆-alkenyl, C₂-C₆-alkynyl or C₁-C₄-alkoxy-C₁-C₂-alkyl,
Xis F or Cl and
n is 0, 1, 2 or 3;
which comprises
A) providing a compound of the formula (II) 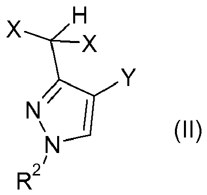 in which
X is F or Cl,
Y is Cl or Br and
R² has one of the meanings given above; and
B) reacting a compound of the formula (II) with carbon monoxide and a compound of the formula (III), 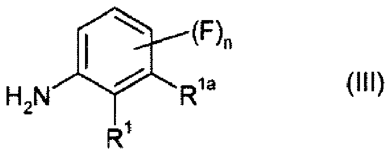 in which R¹, R^{1a} and n have one of the meanings given above,
in the presence of a palladium catalyst.

2. The process according to claim 1 in which the compound of the formula (I) is selected from among compounds of the formula (I.1) 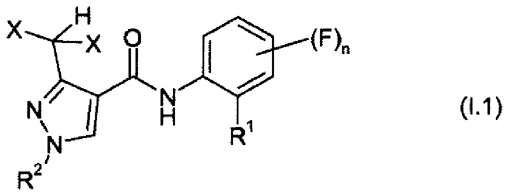 in which R¹, R², X and n independently of one another have one of the meanings given for the compounds of the formula (I),
and in which the compound of the formula (III) is selected from among compounds of the formula (III.1) 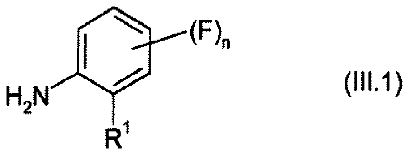 in which R¹ and n independently of one another have one of the meanings given for the compounds of the formula (III).

3. The process according to either of claims 1 and 2 in which the provision of a compound of the formula (II) in which Y represents Br or Cl comprises
A.1) providing a compound of the formula (IV) 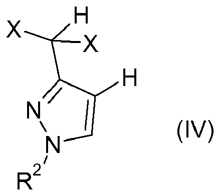 in which
X represents chlorine or fluorine and
R² has one of the meanings given above,
A.2) if appropriate, in the case that X in the compound of the formula (IV) is chlorine, a halogen exchange for fluorine and
A.3) chlorinating or brominating a compound of the formula (IV).

4. The process according to claim 3 in which the provision of a compound of the formula (IV) comprises
A.1.1a) providing a compound of the formula (V) 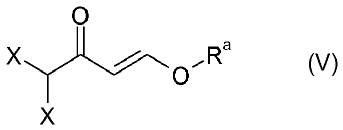 in which
X is Cl or fluorine and
R^{a} is C₁-C₆-alkyl or C₂-C₆-alkenyl, and
A.1.2a) reacting a compound of the formula (V) with a hydrazine compound of the formula R²HN-NH₂ in which R² has one of the meanings given above.

5. The process according to claim 3 in which the provision of a compound of the formula (IV) comprises
A.1.1b) providing a compound of the formula (VI) 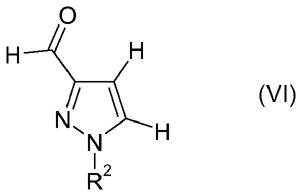 in which R² has one of the meanings given above; and also
A.1.2b) reacting a compound of the formula (VI) by converting the carbonyl group into a dihalomethyl group using a suitable halogenating agent.

6. The process according to claim 3 in which the provision of a compound of the formula (IV) comprises
A.1.1c) deprotonating a propargylaldehyde acetal,
A.1.2c) reacting the deprotonated propargylaldehyde acetal from step A.1.1c with a compound of the formula CHX₂Cl in which X is F or Cl, and
A.1.3c) subsequently converting the reaction product from step A.1.2c into a compound of the formula (IV) using a hydrazine compound of the formula R²HN-NH₂ in which R² has one of the meanings given above.

7. The process according to claim 3 in which the provision of a compound of the formula (IV) in which X is fluorine comprises
A.1.1d) providing a compound of the formula (VII) 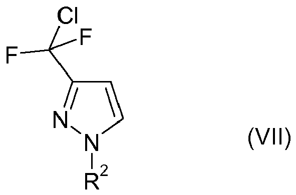 in which R² has one of the meanings given above; and
A.1.2d) dechlorinating the compound of the formula (VII).

8. The process according to claim 3 in which the provision of a compound of the formula (IV) comprises
A.1.1e) providing a compound of the formula (VIII) 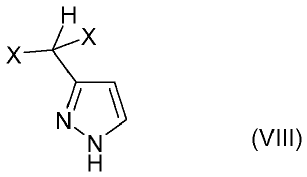 in which X is Cl or fluorine and
A.1.2e) N-alkylating the compound of the formula (VIII).

9. The process according to claim 8 in which the provision of a compound of the formula (VIII) in which X is F comprises
providing a compound of the formula (IX) 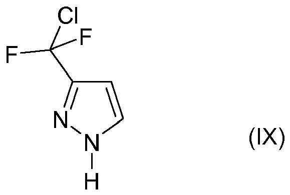 and also dechlorinating the compound of the formula (IX).

10. The process according to either of claims 1 and 2 in which the provision of a compound of the formula (II) comprises
A.1') providing a compound of the formula (VI) as defined in claim 5,
A.2') chlorinating or brominating a compound of the formula (VI) in the 4-position of the pyrazole, giving a compound of the formula (X) 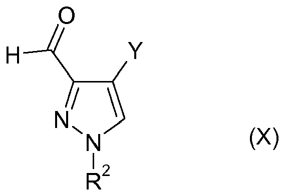 in which Y is Cl or Br, and also
A.3') converting the carbonyl group of the compound of the formula (X) into a dihalomethyl group.

11. The process according to either of claims 1 and 2 in which the provision of a compound of the formula (II) in which Y is Cl comprises A.1") providing a compound of the formula (XI) 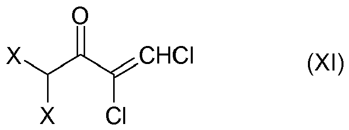 in which X is F or chlorine,
A.2") reacting a compound of the formula (XI) with a suitable hydrazine derivative of the formula R²HN-NH₂ to give a compound of the general formula (II) in which X is F or Cl, Y is Cl and R² has one of the meanings given above, and also
A.3") if appropriate, in the case that X in the compound of the formula (II) is chlorine, a halogen exchange for fluorine.

12. The process according to any of the preceding claims in which X in the compounds of the general formula (I) is fluorine.

13. A compound of the general formula (II) as defined in claim 1.

14. A compound of the general formula (IV) as defined in claim 3.

15. A compound of the general formula (VII) as defined in claim 7.

16. A compound of the general formula (VIII) as defined in claim 8.

17. A compound of the general formula (IX) as defined in claim 9.

18. A process for preparing compounds of the formula (II) as defined in claim 13 which comprises
A.1) providing a compound of the formula (IV) 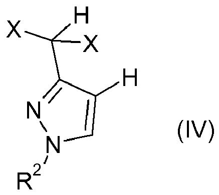 in which
X is chlorine or fluorine and
R² has one of the meanings given above,
A.2) if appropriate, in the case that X in the compound of the formula (IV) is chlorine, a halogen exchange for fluorine and
A.3) chlorinating or brominating a compound of the formula (IV).

## Revendications

1. Procédé pour la préparation de composés de formule (I) 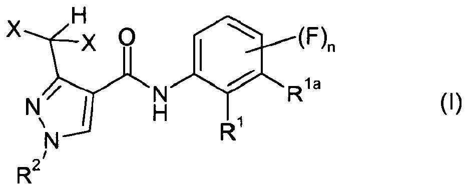 où
R¹ représente phényle ou C₃-C₇-cycloalkyle, qui sont non substitués ou qui présentent 1, 2 ou 3 substituants R^{a1}, qui sont choisis, indépendamment l'un de l'autre, parmi halogène, C₁-C₈-alkyle, C₁-C₄-alcoxy, C₁-C₄-alkylthio, C₁-C₄-halogénoalkyle, C₁-C₄-halogénoalcoxy, C₁-C₄-halogénoalkylthio et C₃-C₇-cycloalkyle,
R^{1a} représente hydrogène ou fluor, ou R^{1a}, ensemble avec R¹, représente C₃-C₅-alcanediyle ou C₅-C₇-cycloalcanediyle, C₃-C₅-alcanediyle et C₅-C₇-cycloalcanediyle étant non substitués ou présentant 1, 2 ou 3 substituants choisis parmi C₁-C₄-alkyle,
R² représente hydrogène, C₁-C₆-alkyle, C₂-C₆-alcényle, C₂-C₆-alcynyle ou C₁-C₄-alcoxy-C₁-C₂-alkyle,
X représente F ou Cl, et
n représente 0, 1, 2 ou 3 ;
comprenant
A) la préparation d'un composé de formule (II) 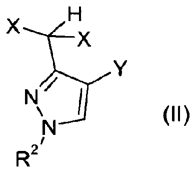 où
X représente F ou Cl,
Y représente Cl ou Br et
R² présente une des significations susmentionnées ;
et
B) la transformation d'un composé de formule (II) avec du monoxyde de carbone et un composé de formule (III), 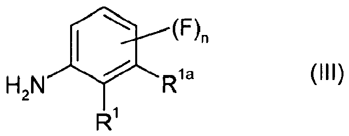 dans laquelle R¹, R^{1a} et n présentent une des significations susmentionnées, en présence d'un catalyseur à base de palladium.

2. Procédé selon la revendication 1, le composé de formule (I) étant choisi parmi les composés de formule (I.1), 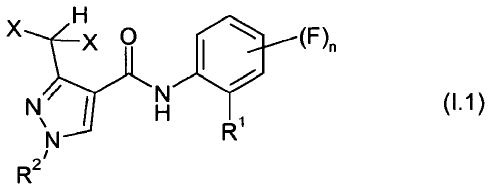 où R¹, R², X et n présentent, indépendamment les uns des autres, une des significations indiquées pour les composés de formule (I),
et où le composé de formule (III) est choisi parmi les composés de formule (III.1), 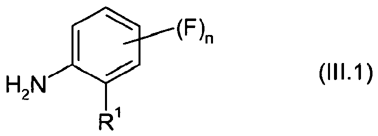 où R¹ et n présentent, indépendamment l'un de l'autre, une des significations indiquées pour les composés de formule (III).

3. Procédé selon l'une quelconque des revendications 1 ou 2, où la préparation d'un composé de formule (II), dans laquelle Y représente Br ou Cl, comprend
A.1) la préparation d'un composé de formule (IV) 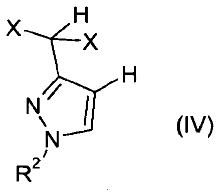 où
X représente chlore ou fluor et
R² présente une des significations susmentionnées,
A.2) le cas échéant, pour le cas où X dans le composé de formule (IV) représente chlore, un échange d'halogène par le fluor et
A.3) la chloration ou la bromuration d'un composé de formule (IV).

4. Procédé selon la revendication 3, où la préparation d'un composé de formule (IV) comprend
A.1.1a) la préparation d'un composé de formule (V) 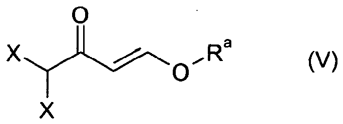 où
X représente Cl ou fluor, et
R^{a} représente C₁-C₆-alkyle ou C₂-C₆-alcényle, et
A.1.2a) la transformation d'un composé de formule (V) avec un composé de type hydrazine de formule R²HN-NH₂, dans laquelle R² présente une des significations indiquées ci-dessus.

5. Procédé selon la revendication 3, où la préparation d'un composé de formule (IV) comprend
A.1.1b) la préparation d'un composé de formule (VI) 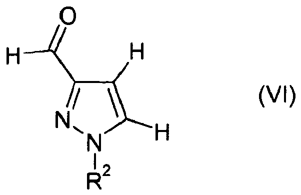 où R² présente une des significations susmentionnées ; ainsi que
A.1.2b) la transformation d'un composé de formule (VI) par transformation du groupe carbonyle en un groupe dihalogénométhyle à l'aide d'un agent d'halogénation appropriée.

6. Procédé selon la revendication 3, où la préparation d'un composé de formule (IV) comprend
A.1.1c) la déprotonation d'un acétal de propargylaldéhyde,
A.1.2c) une transformation de l'acétal de propargylaldéhyde déprotoné de l'étape A.1.1c avec un composé de formule CHX₂Cl, dans laquelle X représente F ou Cl, et
A.1.3c) la transformation consécutive du produit de transformation de l'étape A.1.2c en un composé de formule (IV) avec un composé de type hydrazine de formule R²HN-NH₂, dans laquelle R² présente une des significations indiquées ci-dessus.

7. Procédé selon la revendication 3, où la préparation d'un composé de formule (IV), dans laquelle X représente fluor, comprend
A.1.1d) la préparation d'un composé de formule (VII) 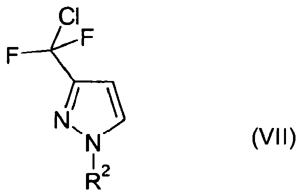 où R² présente une des significations susmentionnées ; et
A.1.2d) la déchloration du composé de formule (VII).

8. Procédé selon la revendication 3, où la préparation d'un composé de formule (IV) comprend
A.1.1e) la préparation d'un composé de formule (VIII) 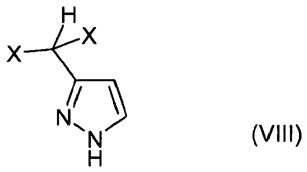 dans laquelle X représente Cl ou fluor, et
A.1.2e) la N-alkylation du composé de formule (VIII).

9. Procédé selon la revendication 8, où la préparation d'un composé de formule (VIII), dans laquelle X représente F comprend
la préparation d'un composé de formule (IX) 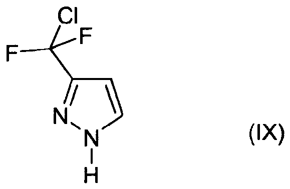 ainsi que la déchloration du composé de formule (IX).

10. Procédé selon l'une quelconque des revendications 1 ou 2, où la préparation d'un composé de formule (II) comprend
A.1') la préparation d'un composé de formule (VI), tel que défini dans la revendication 5,
A.2') la chloration ou la bromuration d'un composé de formule (VI) en 4ème position du pyrazole, avec obtention d'un composé de formule (X), 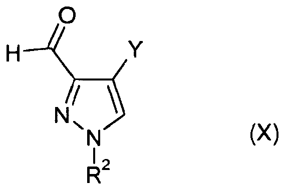 dans laquelle Y représente Cl ou Br, ainsi que
A.3') la transformation du groupe carbonyle du composé de formule (X) en un groupe dihalogénométhyle.

11. Procédé selon l'une quelconque des revendications 1 ou 2, où la préparation d'un composé de formule (II), dans laquelle Y représente Cl, comprend
A.1") la préparation d'un composé de formule (XI) 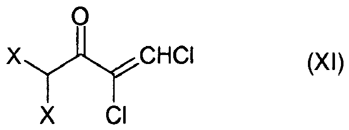 dans laquelle X représente F ou chlore,
A.2") la transformation d'un composé de formule (XI) avec un dérivé d'hydrazine approprié de formule R²HN-NH₂ en un composé de formule générale (II), dans laquelle X représente F ou Cl, Y représente Cl et R² présente une des significations susmentionnées, ainsi que
A.3") le cas échéant, pour le cas où X dans le composé de formule (II) représente chlore, un échange d'halogène par le fluor.

12. Procédé selon l'une quelconque des revendications précédentes, où X, dans les composés de formule générale (I), représente fluor.

13. Composés de formule générale (II) tels que définis dans la revendication 1.

14. Composés de formule générale (IV) tels que définis dans la revendication 3.

15. Composés de formule générale (VII) tels que définis dans la revendication 7.

16. Composés de formule générale (VIII) tels que définis dans la revendication 8.

17. Composés de formule générale (IX) tels que définis dans la revendication 9.

18. Procédé pour la préparation de composés de formule (II) tels que définis dans la revendication 13, comprenant
A.1) la préparation d'un composé de formule (IV) 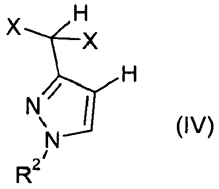 où
X représente chlore ou fluor et
R² présente une des significations susmentionnées,
A.2) le cas échéant, pour le cas où X dans le composé de formule (IV) représente chlore, un échange d'halogène par le fluor et
A.3) la chloration ou la bromuration d'un composé de formule (IV).
